# EUROPEAN PATENT SPECIFICATION

(11) **EP 2 915 880 B1**
(45) Date of publication and mention of the grant of the patent: **28.11.2018**
(21) Application number: 13851635.6
(22) Date of filing: 30.10.2013
(51) Int. Cl.: C12N 5/0783, C12N 5/10, A61K 35/17, A61P 31/04, A61P 31/10, C12N 15/00, C12N 15/09

(54) **MAIT-LIKE CELLS AND METHOD FOR MANUFACTURING SAME**
MAIT-ZELLEN UND VERFAHREN ZUR HERSTELLUNG DAVON
CELLULES DE TYPE MAIT ET LEUR MÉTHODE DE FABRICATION

(30) Priority: 30.10.2012 JP 2012239195
(43) Date of publication of application: 09.09.2015
(73) Proprietor: Daiichi Sankyo Company, Limited, Tokyo 103-8426 (JP); National University Corporation Hokkaido University, Sapporo-shi, Hokkaido 060-0808 (JP)
(72) Inventor: WAKAO, Hiroshi, Sapporo-shi Hokkaido 060-0808 (JP); FUJITA, Hiroyoshi, Sapporo-shi Hokkaido 060-0808 (JP); KOSHIMIZU, Uichi, Kobe-shi Hyogo 650-0047 (JP); YOSHIKIYO, Kazunori, Kobe-shi Hyogo 650-0047 (JP)
(74) Representative: Vossius & Partner Patentanwälte Rechtsanwälte mbB
(86) International application number: PCT/JP2013/080359
(87) International publication number: WO 2014/069672

(56) References cited:
- EP-A1- 2 083 076
- EP-A1- 2 336 303
- EP-A1- 2 434 012
- EP-A1- 2 639 297
- WO-A1-2008/038579
- WO-A1-2010/027094
- WO-A1-2010/134526
- WO-A1-2012/063817
- SUGIMOTO CH ET AL: "Mucosal-associated invariant T cells from induced pluripotent stem cells: A novel approach for modeling human diseases", WORLD JOURNAL OF STEM CELLS, vol. 8, no. 4, April 2016 (2016-04), pages 158-169, XP55273198, ISSN: 1948-0210, DOI: 10.4252/wjsc.v8.i4.158
- LE BOURHIS, L. ET AL.: 'Antimicrobial activity of mucosal-associated invariant T cells' NAT. IMMUNOL. vol. 11, no. 8, August 2010, PAGES 701 - 708, 055243690
- LE BOURHIS, L. ET AL.: 'Mucosal-associated invariant T cells: unconventional development and function' TRENDS IN IMMUNOLOGY vol. 32, no. 5, 2011, pages 212 - 218, XP028202787
- SACHIKO MIYAKE: 'MAIT Cells and Autoimmunity' SAISHIN IGAKU vol. 66, no. 12, 10 December 2011, pages 2736 - 2740, XP008178721
- DUSSEAUX, M. ET AL.: 'Human MAIT cells are xenobiotic-resistant, tissue-targeted, CD161hi IL-17 -secreting T cells' BLOOD vol. 117, no. 4, January 2011, pages 1250 - 1259, XP055243699
- NISHIMURA, K. ET AL.: 'Development of Defective and Persistent Sendai Virus Vector' A UNIQUE GENE DELIVERY/EXPRESSION SYSTEM IDEAL FOR CELL REPROGRAMMING, THE JOURNAL OF BIOLOGICAL CHEMISTRY vol. 286, no. 6, 11 February 2011, pages 4760 - 4771, XP055243702
- KAZUMI TOKORO ET AL.: 'Yakko Yakuri Shiken no Shiken Keikakusho -Shinraisei no Kanten kara' JPN. PHARMACOL. THER. (BASIC PHARMACOLOGY & THERAPEUTICS) vol. 31, no. 3, 2003, pages 196 - 200, XP008178719
- KEIJI YAMAMOTO: 'Anzensei Yakuri Shiken' FOLIA PHARMACOL. JPN. vol. 130, 2007, pages 299 - 303, XP008178743
- WAKAO, H. ET AL.: 'Expansion of Functional Human Mucosal-Associated Invariant T Cells via Reprogramming to Pluripotency and Redifferentiation' CELL STEM CELL vol. 12, 02 May 2013, pages 546 - 558, XP055243716

## Description

### TECHNICAL FIELD

The present invention relates to a method for preparing induced pluripotent stem cells from MAIT cells, and induced pluripotent stem cells derived from MAIT cells. The present invention further relates to a method for preparing MAIT-like cells from induced pluripotent stem cells and also to MAIT-like cells obtained thereby.

### BACKGROUND ART

MAIT cells (Mucosal associated invariant T cells) are a type of innate T-lymphocytes, which are known as cells that produce different cytokines to regulate various immune responses and that act as a "bridge" between innate immunity and adaptive immunity. MAIT cells exist abundantly in humans, occupying, for example, 20-50% among T cells in liver and 1-10% among lamina propria lymphocytes (LPL) or peripheral blood mononuclear cells (PBMC), but they are rare in mice (Dusseaux et al., 2011; Le Bourhis et al., 2011).

Another feature of MAIT cells is the invariance of the T cell receptor (TCR). TCRs, which are specifically expressed in T cells, recognize the peptide fragment bound to a major histocompatibility complex (MHC) molecule as an antigen. A TCR is composed of two polypeptide chains expressed on the cell surface, the α chain and the β chain, and these chains both have a variable (V) region at the antigen binding region on the N terminal, and the amino acid sequence of this site differs for each TCR molecule, providing variety in the V region, which accounts for the antigen specificity of T cells in immune reactions. A V region of a TCR consists of V (Variable), D (Diversity), J (Joining) gene fragments having many subtypes, like as immunoglobulins. The gene encoding the region is formed by DNA recombination of a single fragment of each of V and J in the α chain, and DNA recombination of a single fragment of each of V, D, and J in the β chain.

Meanwhile, there are two types of T cells known to have an invariant type of TCR, namely natural killer T cells (NKT cells) and MAIT cells. The TCR α chain of the human MAIT cell takes the combination of Vα 7.2-Jα 33 only, and the NKT cell takes the combination of Vα 24-Jα 18 (Le Bourhis et al., 2011). The TCR α chain of the mouse MAIT cell takes the combination of Vα 19-Jα 33 only, and the NKT cell takes the combination of Vα 14-Jα 18. The TCRs of the two cells also differ in the antigen-presenting molecules they can bind to (restriction): whereas the TCR of the MAIT cell recognizes/binds to MR1 (MHC-related molecule-1), which has no variation and is similar to MHC, an antigen-presenting molecule in general T cells, the antigen-presenting molecule of NKT is CD1d (cluster of differentiation-I d), which is similar to MHC. The TCR specific to MAIT cells and MR1 are evolutionarily conserved well, and their functional importance for a wide range of species is suggested (Le Bourhis et al., 2011).

Not only does the MAIT cell express the above invariant TCR α chain, it also expresses a C-type lectin CD161 (also known as natural killer cell surface protein: NKRP1) and interleukin (IL)-18 receptor α chain (IL-18Rα) as specific surface antigen marker molecules (Cosmi et al., 2008; Le Bourhis et al., 2010). In other words, the MAIT cell can be defined as a cell that expresses a MAIT-specific invariant TCR α chain, CD161, and IL-18Rα together with general T cell markers, such as CD3. Further, since the MAIT cells express the character of effecter/memory T cells, such as CD45RA⁻, CD45RO⁺, CD95^{high}, CD62L^{low}, and the chemokine receptor is expressed as CCR9^{int}, CCR7⁻, CCR5^{high}, CXCR6^{high}, CCR6^{high}, it can be inferred that the MAIT cells are directed to homing to the intestines, liver, etc. (Dusseaux et al., 2011).

Further, the MAIT cells are reported as producing granzyme B or cytokines, such as interferon (IFN) γ, IL-17, IL-2, and as exhibiting almost no proliferation ability, but exhibiting multidrug-resistance by expressing a multidrug-resistant carrier ABCB1 (Dusseaux et al., 2011). These features suggest that the MAIT cells are resistant to foreign matters produced by the enteric bacteria, and/or are involved with the defense system against infections in the living body. In fact, in a report that MAIT cells are special T cells that react with cells infected with bacteria, such as tubercle bacillus, or fungus, it was also indicated that they decreased in patients infected with bacteria, such as tuberculosis, etc., and that they protected against infection by Mycobacterium abscessus or Escherichia coli, as shown by a model experiment using mice (Le Bourhis et al., 2010; Gold et al., 2010; Dusseaux et al., 2011). It is thus presumed that the MAIT cells function as innate lymphocytes against bacterial infection.

In addition, it has been suggested that the MAIT cells are involved in multiple sclerosis and other autoimmune diseases, inflammatory diseases, and the onset/development of cancer. The CD8⁺/CD161^{high} T cells are a T cell group that accumulates at inflammatory sites including the liver, joints, etc., and that is seen as the cause of crisis of multiple sclerosis. Ninety percent of such CD8⁺/CD161^{high} T cells in the human PBMC comprises MAIT TCRα-specific Vα7.2⁺cells (Walker et al., 2011). Further, the accumulation of MAIT cells is even greater at the legion site of a multiple sclerosis patient (Illes et al., 2004; Miyazaki et al., 2011). Reports on the accumulation of MAIT cells also exist for renal tumor or brain tumor (Peterfalvi et al., 2008), chronic inflammatory demyelinating polyneuropathy (Illes et al., 2004). It is also reported concerning inflammatory bowel diseases represented by ulcerative colitis or Crohn's disease that the transferred MAIT cells act protectively against the inflammatory tissue injury evoked by a pharmaceutical agent (Xiao Ruijing et al., 2012).

As such, the MAIT cell is suggested as being involved in various diseases and pathological conditions, but no sufficient progress has been made so far in the study/analysis of the immunity control mechanism, particularly the action and specific mechanism in immune immunity, factors and molecules contributing thereto, and its significance in the onset/development of diseases. One major reason is the problem of the cell source that can be provided for in vitro and in vivo studies.

As shown above, MAIT cells are an extremely rare cell group in mice frequently used as the experimental animal, and a functional analysis using said animal is difficult. Meanwhile, MAIT cells exist more abundantly in human compared to mice, but a preparation of large amounts of MAIT cells from human biological samples, such as peripheral blood, is restricted. Further, such method holds a high possibility that the number and characteristic of the obtained MAIT cells would fluctuate largely, and the stability and reproducibility of a test using such cells are deficient. Further, the MAIT cells are normally in a state in which it has almost no cell proliferation ability, moreover it cannot be expanded under an in vitro condition, since the factor or stimulus that induces proliferation is not identified (Dusseaux et al., 2011). One solution to widely advance researches using MAIT cells may be, for example, to use a model cell having a similar function to that of the MAIT cell, but almost no cell line having such feature is currently known.

Further, one possible future application of the MAIT cell is a use as a cell source in the so-called cell transplantation treatment, which is a treatment that transfers the MAIT cell or an artificially modified MAIT cell to patients with different infectious diseases or autoimmune diseases or cancer. However, to implement such treatment, it is essential to establish a method of preparing a large amount of MAIT cells having a stable quality.

Recently, various attempts are made in the method of preparing different cells in vitro to create target cells by inducing differentiation of pluripotent stem cells, used as the original cell. Pluripotent stem cells are defined as cells that can be proliferated by cultivation in a test tube almost eternally or for a long time while maintaining an undifferentiated state, that exhibit a normal nucleus (chromosome) type, and that are capable of differentiating into cells of all three lineages (ectoderm, mesoderm, and endoderm). Examples of pluripotent stem cells include embryonic stem cells (ES cells) isolated from the early embryo, or embryonic germ cells isolated from primordial germ cells of the fetal period, the germline stem cells isolated from the testis immediately after birth, and additionally, induced pluripotent stem cells (hereinafter referred to as iPS cells) created from somatic cells, such as a fibroblast by special gene manipulation (Lengner, 2010; Pfannkuche et al, 2010; Okita and Yamanaka, 2011).

Many cases are reported of creating hemocyte cells or lymphocyte cells from pluripotent stem cells, and a method for selectively creating T cells is also known. In other words, it is possible to succeed the process of retaining pluripotent stem cells, such as ES cells or iPS cells, in an undifferentiated state, then seeding them on stroma cells having hematopoietic cell maintenance ability (e.g. OP9 cells) to induce differentiation to haematopoietic stem cells and precusor cells, and to recover the differentiated cells by a process of creating cells having T cell characteristics on the OP9 cell (OP9/DLL1) forcefully expressing a Notch ligand, delta-like-1 (DLL1) (Schmitt et al., 2004; Timmermans F et al., 2011). There is also report of a method that subjects iPS cells created from mouse NKT cells to the OP9 coculture system to create cells having NKT cell characteristics (WO 2008/038579; Wakao H et al., 2008; WO 2010/027094; Watarai et al., 2010).

### CITATION LIST

### PATENT DOCUMENTS

Patent Document 1: WO 2008/038579
Patent Document 2: WO 2010/027094

### NON-PATENT DOCUMENTS

Non-Patent Document 1: Dusseaux et al., Blood 117, 1250-1259 (2011)
Non-Patent Document 2: Le Bourhis et al., Trends in Immunol. 32, 212-218 (2011)
Non-Patent Document 3: Cosmi et al., J. Exp. Med. 205, 1903-1916, (2008)
Non-Patent Document 4: Le Bourhis et al., Nat. Immunol. 11, 701-708 (2010)
Non-Patent Document 5: Gold et al., PLoS Biol. 8, e1000407 (2010)
Non-Patent Document 6: Walker et al., Blood 119, 422-433 (2011)
Non-Patent Document 7: Illes et al., Int. Immunol. 16, 223-230 (2004)
Non-Patent Document 8: Miyazaki et al., Int. Immunol. 23, 529-535 (2011)
Non-Patent Document 9: Peterfalvi et al., Int.Immunol. 20, 1517-1525 (2008)
Non-Patent Document 10: Xiao Ruijing et al., Hepatogastroenterology 115, 762-767 (2012)
Non-Patent Document 11: Lengner CJ, Ann. N.Y. Acad. Sci. 1192, 38-44 (2010)
Non-Patent Document 12: Pfannkuche K et al., Cell Physiol Biochem. 26, 105-24 (2010)
Non-Patent Document 13: Okita and Yamanaka, Philos. Trans. R Soc. Lond. B Biol. Sci. 366, 2198-2207 (2011)
Non-Patent Document 14: Schmitt et al., Nat. Immunol. 5, 410-417 (2004)
Non-Patent Document 15: Timmermans et al., J. Immunol. 182, 6879-6888 (2011)
Non-Patent Document 16: Wakao et al., FASEB J. 22, 2223-2231 (2008)
Non-Patent Document 17: Watarai et al., J. Clin. Invest. 120, 2610-8 (2010)

### SUMMARY OF INVENTION

### TECHNICAL PROBLEM

T cells known for having a single invariant TCR are NKT cells and MAIT cells. The important role that NKT cells play in protecting an individual against bacteria and cancer, as well as their involvement in the pathology of an autoimmune disease was made known mainly by a study using mice. Meanwhile, MAIT cells exist in large numbers in the human peripheral blood, intestines, or liver, and they play an important role in the mucosa immunity, but much of the detail is still not elucidated.

Conventionally, functional analysis using mainly mice has been carried out to link NKT cells to innovative drug development with focus on the immunology control ability of NKT cells, but it was frequently the case that the analysis results of mice differed from those of human. NKT cells exist relatively abundantly in mice, but are extremely rare in human, and many attempts of innovative drug development targeting human NKT cells have ended in failure.

In comparison, MAIT cells are extremely rare in mice, but are known to exist abundantly in human. Since NKT cells and MAIT cells have similar specific characters, a view that the human MAIT cell has a similar function as the mouse NKT cell, in other words, that the human MAIT cell is the functional cell corresponding to the mouse NKT cell, has been presented. Accordingly, it is extremely important to advance the functional analysis of the MAIT cell, and apply the result to innovative drug development.

However, the MAIT cell does not react to any T cell proliferation stimulus known to date, and it has been difficult to prepare a large amount of MAIT cells required in a functional analysis. In particular, MAIT cells are an extremely rare cell group in mice frequently used as experimental animals, and the pursuit of research and development using mouse MAIT cells has limitations. Further, although abundant MAIT cells exist in human, preparation of a large amount of MAIT cells that depends on their preparation from a human living body has limitations, since MAIT cells are difficult to proliferate. As shown above, MAIT cells must be obtained by preparation from a living body and purification, and no method for inducing differentiation in vitro /amplification nor technology relating to character analogous cells (model cells) exist.

Accordingly, an object of the present invention is to establish MAIT-like cells having a similar function as MAIT cells and to establish a technology for creating such cells. Another object of the present invention is providing a method for creating induced pluripotent stem cells from MAIT cells, and the induced pluripotent stem cells derived from MAIT cells.

### SOLUTION TO PROBLEM

The present inventors performed extensive research to solve the above problem, and was successful in creating induced pluripotent stem cells (iPS cells) derived from MAIT cells by reprogramming MAIT cells, and further was successful in obtaining MAIT-like cells by inducing differentiation of induced pluripotent stem cells derived from MAIT cells.

The present invention encompasses the following inventions without being limited thereby.
(1) A preparation method of a MAIT-like cell comprising:
   introducing a reprogramming factor to a MAIT cell to obtain an induced pluripotent stem cell retaining a TCR α chain gene rearranged to a MAIT cell-specific fashon; then
   inducing differentiation of the induced pluripotent stem cell to obtain a MAIT-like cell.
(2) A preparation method of an induced pluripotent stem cell comprising:
   introducing a reprogramming factor to a MAIT cell to obtain an induced pluripotent stem cell retaining a TCR α chain gene rearranged to a MAIT cell-specific fashion.
(3) The method according to either (1) or (2), wherein the reprogramming factor is introduced using a virus vector.
(4) The method according to (3), wherein the virus vector is Sendai virus vector.
(5) The method according to (4), wherein the Sendai virus vector incorporates a plurality of reprogramming factors in a same vector.
(6) An induced pluripotent stem cell retaining a TCR α chain gene rearranged to a MAIT cell-specific fashion.
(7) The induced pluripotent stem cell according to (6) retaining only a single TCR α chain gene rearranged to a MAIT cell-specific fashion as a TCR α chain gene.
(8) The induced pluripotent stem cell according to either (6) or (7), wherein the TCR α chain gene rearranged to a MAIT cell-specific fashion is Vα 7.2-Jα 33 for a human and Vα 19-Jα 33 for a mouse.
(9) The induced pluripotent stem cell according to any one of (6) to (8) obtained by any method of (2) to (5).
(10) A preparation method of a MAIT-like cell comprising:
   inducing differentiation of the induced pluripotent stem cell according to any one of (6) to (9) to obtain a MAIT-like cell.
(11) The method according to (10) comprising co-cultivating the induced pluripotent stem cell with a feeder cell to obtain a MAIT-like cell.
(12) A MAIT-like cell obtained by the method according to either (10) or (11).
(13) The MAIT-like cell according to (12) exhibiting a positive expression of CD45RA.
(14) An evaluation method of an activity to regulate a MAIT cell function of a test substance comprising a step of bringing the MAIT-like cell according to any of (12) or (13) with the test substance.
(15) A cell therapy agent containing the MAIT-like cell according to any of (12) or (13).
(16) The cell therapy agent according to (15) that is administered to improve resistance against bacterial infection or fungal infection.

### ADVANTAGEOUS EFFECTS OF INVENTION

The present invention enables induced pluripotent stem cells to be created from MAIT cells. Further, the present invention enables MAIT-like cells to be created from induced pluripotent stem cells.

### BRIEF DESCRIPTION OF DRAWINGS

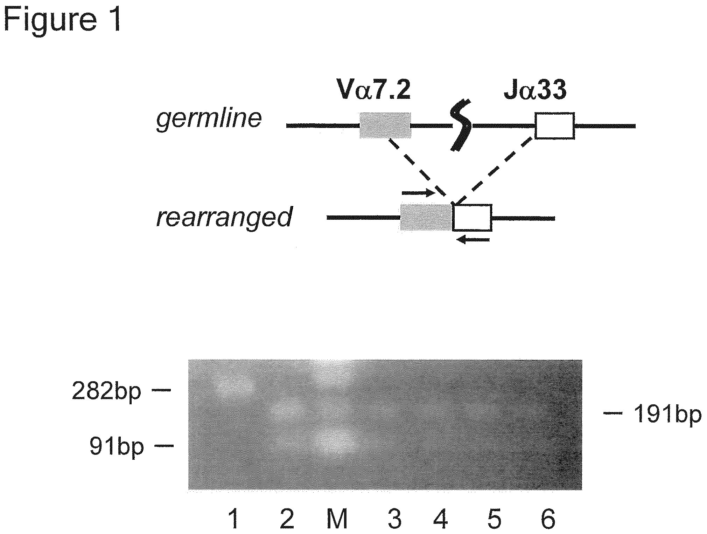

The top column of Figure 1 is a diagram showing the gene rearrangement form of the TCR α chain in a MAIT cell. Gene sequences encoding the Vα 7.2 and Jα 33 regions at distant locations on the same chromosome in the germline genome are rearranged on the MAIT cell to encode a single continuous gene. The arrow illustrates the positions of the primers of SEQ NO. 1 and 2 in Example 1. In addition, the bottom column of Figure 1 shows a result of studying the rearrangement of the Vα 7.2 and Jα 33 regions in the 5 iPS-like cell lines established from MAIT cells prepared from human cord blood cells. The genome DNAs extracted from the cell lines were used as templates to perform PCR reaction with primers of SEQ ID NO: 1 and 2 of Example 1. The PCR products were digested with a restriction endonucleases SacI and separated on a 2% agarose gel, and stained with ethidium bromide. 1: PCR productderived from an iPS-like cell line (1-3D) created from a MAIT cell (not digested by SacI), 2: PCR product derived from 1-3D line digested by SacI, M: DNA size marker, 3-6: PCR products derived from iPS-like cell lines created from MAIT cells, and digested by SacI.

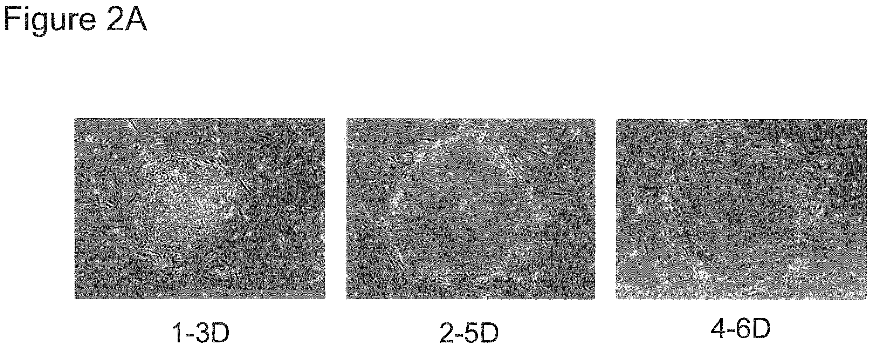

Figure 2A is photographs of iPS cells established from MAIT cells prepared from human cord blood cells.

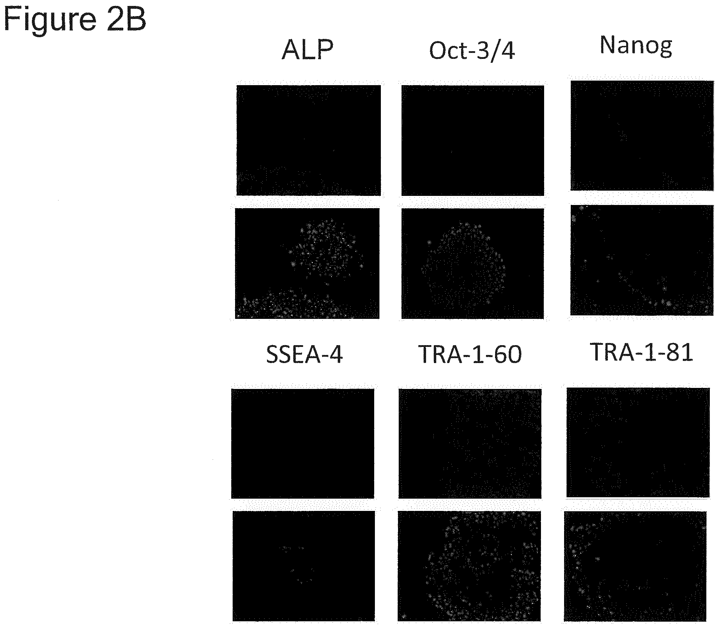

Figure 2B is staining images showing the expression of different ES/iPS-cell-specific markers in iPS cells established from a human MAIT cell (MAIT-iPS cell: 1-3D line). The top column is immunostained images obtained using a specific antibody, and the bottom column is nucleus staining images by DAPI (4',6-diamidino-2-phenyl indole).

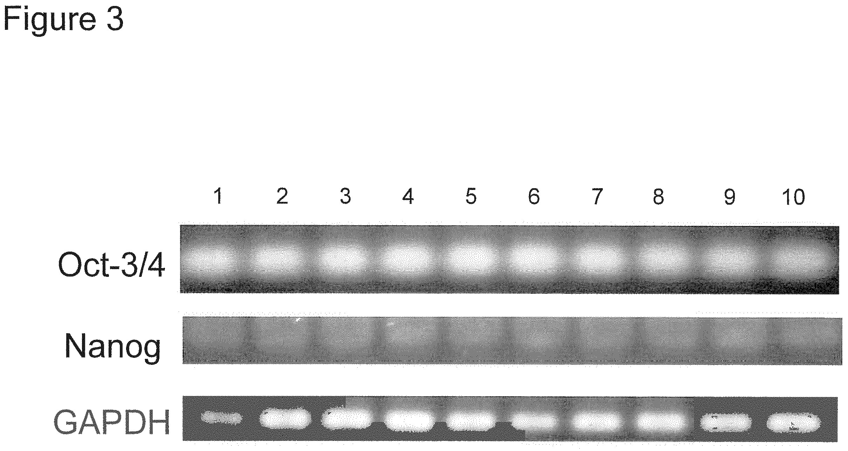

Figure 3 is electrophoresis pictures showing specific gene expressions in MAIT-iPS. 1: human iPS cell (line B7), 2: human MAIT-iPS cell (line 1-3D), 3: human MAIT-iPS cell (line 2-5D), human MAIT-iPS cell (line 4-6D), human MAIT-iPS cell (line A11), human MAIT-iPS cell (line A13), human MAIT-iPS cell (line A46), human MAIT-iPS cell (line C4B-), human MAIT-iPS cell (line C5B), human MAIT-iPS cell (line C7I).

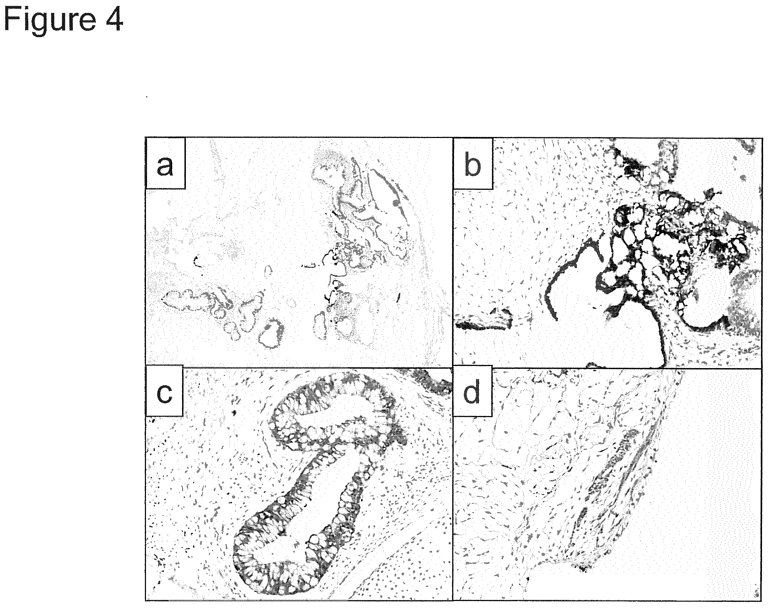

Figure 4 is histological images of a teratoma created from the MAIT-iPS cell. a: the full image (magnification: ×40), b: neural tube like structure containing melanin positive cells (the black cells in the diagram) (magnification: ×200), c: intestine like structure arranged by the keratin positive epithelial cell (magnification: ×200), d: musculature like structure that is desmin positive (magnification: ×200).

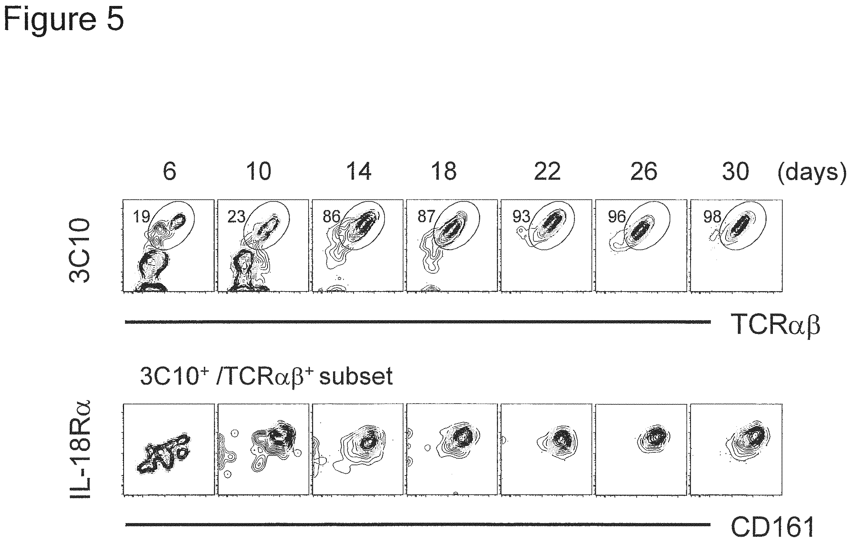

Figure 5 is a diagram showing the different MAIT cell marker expressions in cells created by inducing differentiation of MAIT-iPS cells. The numbers are the number of days from inducing differentiation to T cell lineage cells. The top column shows reactivity to an anti-TCR Vα 7.2 antibody (3C10) and an anti-TCR αβ antibody (IP26), and the part in the diagram surrounded by a solid line is a double-positive fraction (3C10⁺/TCRαβ⁺ cell), and its percentage ratio against the whole is shown. The bottom column shows the reactivity against the anti-IL-18 Rα antibody (H44) and anti-CD161 antibody (DX12) concerning the double-positive fraction.

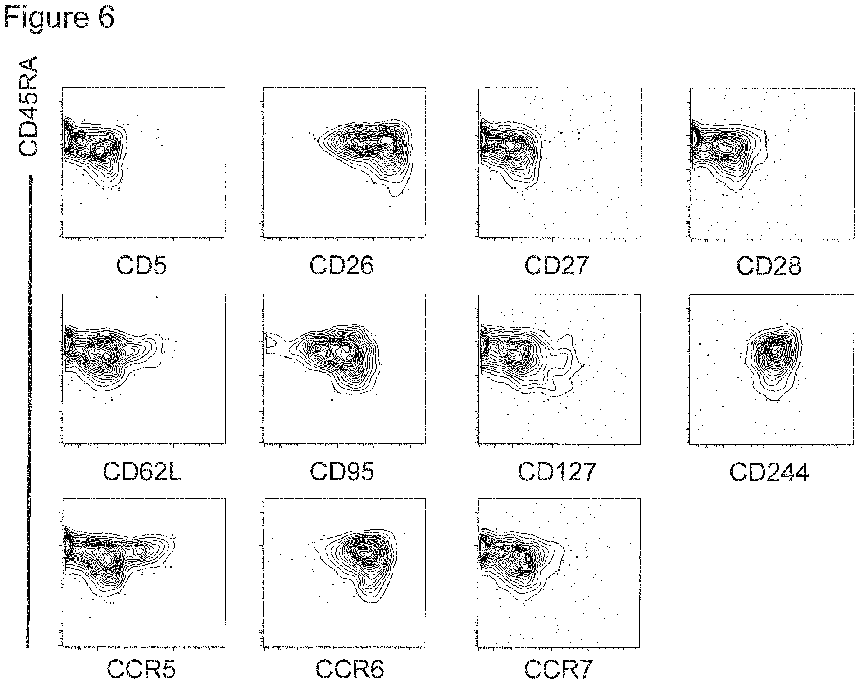

Figure 6 is a diagram showing the expression forms of the different surface antigen markers in the iMAIT cell.

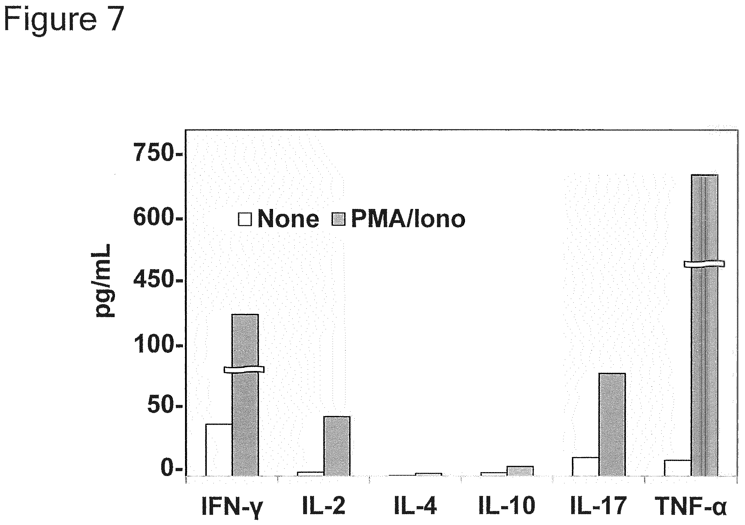

Figure 7 is a diagram showing the different cytokine production abilities of the iMAIT cells. None: no stimulus, PMA/Iono: PMA/Ionomycin stimulus.

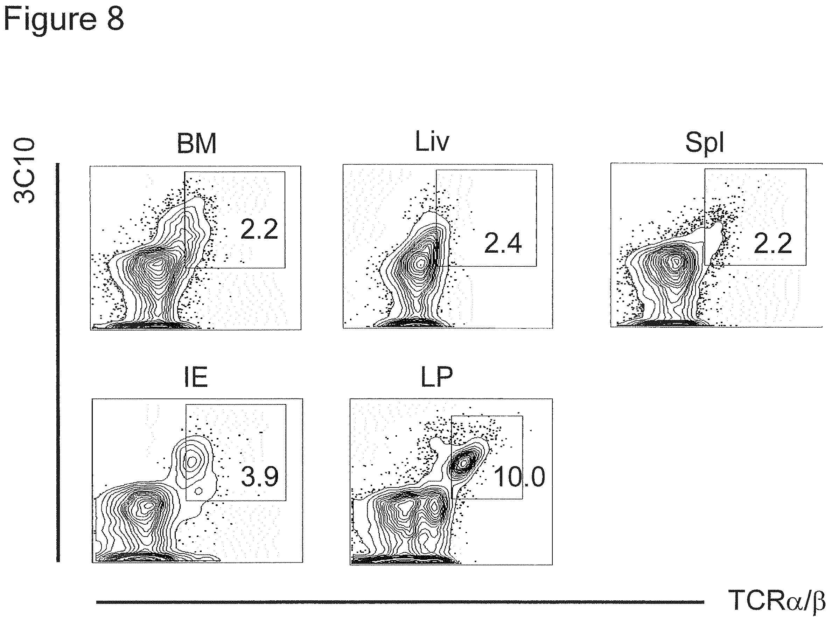

Figure 8 is a diagram showing the presence in different organs of iMAIT cells transplanted in the mouse body. The 3C10⁺/TCRαβ⁺ cell was studied for lymphocytes recovered from different organs. The part surrounded by a solid line in the drawing is the 3C10⁺/TCRαβ⁺ cell, that is, the iMAIT cell, and its percentage ratio against the whole is shown. BM: bone marrow, Liv: liver, Spl: spleen, IE: intestinal epithelia, LP: lamina propria lymphocyte.

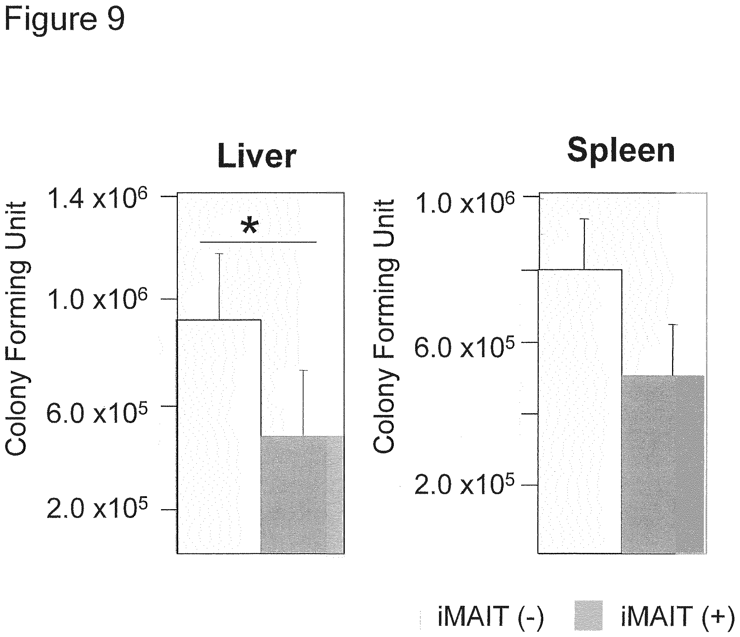

Figure 9 is a result of verifying defense activity of the iMAIT cell. Mouse receiving iMAIT cell transplantation was injected with M. abscessus, and the colony forming units of bacteria in the liver and spleen were measured 2 weeks later.

### DESCRIPTION OF EMBODIMENTS

One aspect of the present invention is a method for creating cells having a MAIT cell-like character (referred to hereinafter as MAIT-like cells), in which a MAIT-like cell can be obtained by introducing a reprogramming factor into the MAIT cell using an expression vector to obtain an induced pluripotent stem cell (referred to hereinafter as iPS cell) derived from a MAIT cell, then inducing differentiation of the iPS cell.

Another aspect of the present invention is to obtain iPS cells by reprogramming MAIT cells. In a preferable aspect, an iPS cell derived from the MAIT cell can be obtained by introducing a reprogramming factor to the MAIT cell using a virus vector, particularly a Sendai virus vector. The iPS cell, in which the TCR α chain gene is rearranged to a single Vα-Jα of a sequence specific to a MAIT cell (MAIT-iPS cell), is a cell having a general iPS characteristic , and it has self-replication ability as well as pluripotency, and it shows a gene expression form similar to the ES cell.

An "iPS cell" in the present invention is a cell that obtained pluripotency and self-replication ability artificially by introducing the reprogramming factors (nucleus reprogramming factors) in the somatic celland inducing its expression, and that has characters similar to the ES cell. "Pluripotency" is defined as a cell having the ability to differentiate into cells of all lines under an appropriate condition, but a cell does not necessarily need to be able to differentiate into cells of all lines when performing the present invention, as long as it has the ability to differentiate into a MAIT cell, a stem cell/precursor cell of the MAIT cell, and at least one other cell line. Characters similar to the ES cell can be defined by the presence of a surface marker molecule specific to ES cells, expression of cell biological characters specific to ES cells, such as the teratoma forming ability, or genes specific to ES cells, or the high similarity in the expression form of many gene groups in the subject cell.

MAIT cells in the present invention are T cells whose TCR α chain gene is rearranged to a unique and uniform Vα-Jα (Vα19-Jα33 in a mouse, Vα7.2-Jα33 in human), and more preferably they can be defined as cells that express CD161 or IL-18Rα. Further, the MAIT cell can be characterized by the fact that its TCR α chain is restricted by the invariant MR1. Further, the MAIT cell in the present invention can be identified by expression of genes specific to the MAIT cell, or by a cell biological characteristic specific to the MAIT cell.

The MAIT cell used in the present invention is not particularly limited by origin, and MAIT cells derived from mammals, such as human, mouse, or monkey, can be preferably used. Further, the MAIT cell in the living body is almost completely lacking proliferation ability, and no technology to proliferate MAIT cells in vitro has been established, so it is necessary to collect MAIT cells from the living body. The site of collection is not particularly limited, and MAIT cells derived, for example, from cord blood, peripheral blood, liver, thymus gland, spleen, bone marrow, and intestines (lamina propria mucosae, Peyer patch) can be preferably used, and MAIT cells derived from peripheral blood or cord blood are particularly preferable in the present invention.

When the iPS cell is created by reprogramming the MAIT cell in the present invention, the reprogramming factor (nucleic reprogramming factor) to be used can be any known reprogramming factor without particular limitation, and it can be arranged from any substance including a protein facter, a nucleic acid encoding the same (including a form incorporated into a vector), or low molecular compounds. For example, four applicable factors known as the Yamanaka factors are the Oct3/4 gene product (nucleotide sequence: SEQ ID NO: 9), Klf family gene products including Klf4 (nucleotide sequence: SEQ ID NO: 10), Myc family gene products including c-Myc (nucleotide sequence: SEQ ID NO: 11) and Sox family gene products including Sox2 (nucleotide sequence: SEQ ID NO: 12). Further, it is known that iPS cells are obtained by introducing the three factors of Oct3/4 gene products, Klf family gene products and Sox family gene products, then cultivating the factors under the presence of basic fibroblast growth factor (bFGF) (international publication WO 2007/69666). Note that family genes that can be preferably used are those having an identity of 80% or higher or 90% or higher.

Further, it is reported that a part of the above factors can be replaced by agents, such as low molecular compounds. For example, cells, to which two genes of Oct3/4 and Sox2 are introduced, can be treated with valproic acid, which is a histone deacetylase inhibitor, to create iPS cells (Huangfu D et al., Nat. Biotechnol. 26, 1269-1275 (2008)). Further, a method using microRNAs instead of the above factors is also publicly known (Miyoshi N et al., Cell Stem Cells 8, 633-638 (2011)).

One method for introducing the reprogramming factor to a MAIT cell is to introduce the above factors as protein, but it is more preferable to use them in the form of nucleic acids (DNA, RNA, DNA/RNA chimera) encoding the same. The nucleic acid (preferably cDNA) is introduced into a plasmid vector or a virus vector that can function in the host MAIT cell to construct an expression vector and to be provided to the nucleus reprogramming step.

Expression vectors can be any vector that allows efficient transcription and expression of the reprogramming factor gene in the MAIT cell and that can induce subsequent reprogramming (creation of iPS cells), and a preferable example in the present invention is a Sendai virus vector (SeV). The Sendai virus is a virus having a single chain non-segmented minus chain RNA as a genome, and it has been used widely in the field of cell biology. The Sendai virus vector is advantageous in that it can introduce genes in cells and tissues of many mammals, and it can leave the host chromosome unaffected, due to the vector genome maintaining the RNA form in the cytoplasm. A kit product for constructing the Sendai virus vector is commercialized, and a person skilled in the art can obtain it as necessary.

Generally speaking, a method of using retrovirus in addition to adenovirus is widely known as a method for efficiently introducing a gene to a culture using a virus vector. Upon studies, the present inventors were able to reprogram MAIT cells and obtain iPS cells with extreme efficiency in the present invention using MAIT cells by using the Sendai virus vector, and they were further able to obtain iPS cells which are extremely safe due to the lack of rearrangement of genomes.

Further, a common process for introducing multiple reprogramming factors is inserting one or a plurality of genes into individual vectors, and treating host cells with the multiple types of vectors simultaneously, but a more preferable process is to use a vector that can express all genes by mounting multiple reprogramming genes on one vector. The "multiple reprogramming factors" are at least 2 factors selected from the 4 factors of Oct3/4 genes, Klf family genes, Myc family genes and Sox family genes, and preferably 3 factors of Oct3/4 genes, Klf family genes and Sox family genes, and more preferably all 4 factors. Such Sendai virus vectors that can simultaneously express multiple reprogramming genes are known, and there are reports of examples, such as SeVdp(MKOS)302L (SEQ ID NO: 13), which is a Sendai virus vector loaded with reprogramming factors starting from the transcription starting position and arranged as c-Myc→Klf4→Oct3/4→Sox2, or SeVdp(KOSM) (SEQ ID NO: 14), which is a Sendai virus vector arranged as Klf4→Oct3/4 →Sox2→c-Myc inducing and establishing iPS cells from a fibroblast, etc. with an extremely high efficiency (WO 2010/134526; Nishimiura K et al., J. Biol. Chem. 286, 4760-4771 (2011), WO 2012/0063817). Further, a Sendai virus vector designed arbitrarily concerning the arrangement, insertion position of reprogramming factors and other additional sequences, based on the knowledge of the above vectors, can be used in the present invention. For example, SeVdp(KOSM)302L used in Example 1 is a Sendai virus vector that is loaded with reprogramming factors in an arrangement of Klf4→Oct3/4→Sox2→c-Myc similarly to SeVdp(KOSM), and adopts features of SeVdp(KOSM) and SeVdp(MKOS)302L concerning the insertion position and other additional sequences.

Accordingly, a reprogramming factor can be introduced in a MAIT cell using a Sendai virus vector, etc. to obtain a MAIT cell-derived iPS cell, and such MAIT-cell-derived iPS cell is itself an aspect of the present invention. The iPS cell obtained by reprogramming the MAIT cell (MAIT-iPS cell) differs from existing pluripotent stem cells, such as the ES cell or iPS cell, in that it expresses a TCR α chain gene specific to a MAIT cell and/or a product of such TCR α chain gene (such feature referred to hereinafter as "having a MAIT cell-specific TCR α chain"). The pluripotent stem cell having a MAIT cell-specific TCR α chain gene is quite useful in that it allows a MAIT-like cell having characteristics similar to the MAIT cell to be selectively obtained when it is put under conditions inducing T cell differentiation.

The MAIT-iPS cell obtained by reprogramming a MAIT cell can subsequently be collected at high purity and a large amount by cell recovery, separation and purification using known methods.

Further, another aspect of the present invention is iPS cells having MAIT cell-specific TCR α chain genes. Such iPS cells can be obtained by the above mentioned method of introducing a reprogramming factor into a MAIT cell using an expression vector, such as the Sendai virus vector. In addition to such method of reprogramming a somatic cell, the MAIT cell, the iPS cells can be obtained by introducing MAIT cell-specific TCR genes to pluripotent stem cells, such as ES cells or iPS cells, created by general, common methods. In such case, pluripotent stem cells that can be used include not just ES cells, but also all pluripotent stem cells having characters similar to ES cells derived from the cell of an adult organ or tissue, the bone marrow cell, the blood cell, and further cells of embryo or a fetus of a mammal. In that case, the characters similar to the ES cell can be defined by the presence of a surface marker molecule specific to ES cells, cell biological features specific to ES cells, such as a teratoma forming ability, a gene expression specific to ES cells, or a high similarity of the expression pattern of many gene groups in the subject cell.

One aspect of the present invention is a production method of MAIT-like cells, in which the MAIT-like cells can be obtained by inducing differentiation of iPS cells having a MAIT cell-specific TCR α chain gene, such as MAIT-iPS cells. The differentiated cells obtained by such method have the same characters as MAIT cells, and is referred to as MAIT-like cells in the present invention. Particularly, the MAIT-like cells obtained by inducing differentiation of MAIT-iPS cells are referred to as iMAIT cells in the present invention (also referred to as "reMAIT cells" academically).

Known methods of inducing differentiation of T cells from iPS cells or other pluripotent stem cells can be used without limitation when obtaining MAIT-like cells in the present invention by inducing differentiation of iPS cells having a MAIT cell-specific TCR α chain gene. There has been reports concerning NKT cells on obtaining NKT-like cells by performing nuclear transplantation of ES cells obtained from the nuclear transplantation of NKT cells, and inducing differentiation of said cells, and further, obtaining NKT-like cells by reprogramming mouse NKT cells using the retro virus vector to create iPS cells, and then inducing differentiation of the iPS cell.

Any cultivation method for obtaining MAIT-like cells by inducing differentiation in the present invention can be used as long as MAIT-like cells can be obtained, and examples include coculture with feeder cells, float culture, hanging-drop culture, gyratory culture, soft agar culture, microcarrier culture. In a preferable aspect of the present invention, it is preferable to obtain MAIT-like cells by inducing differentiation of iPS cells by coculture, and specifically, MAIT-like cells can be efficiently obtained from MAIT-iPS cells by coculture using stroma cells, such as OP9 cells, as feeder cells, followed by coculture with OP9 cells forcefully expressing a Notch ligand DLL1 (OP9/DLL1), or a coculture that is performed with the OP9/DLL1 cells from the beginning.

The MAIT-like cells obtained by such method can be subsequently subjected to cell recovery, separation, and purification by known methods. The MAIT-like cells obtained by the present invention are cells showing morphological, physiological and/or immunological features that are almost equal to those of a MAIT cell in the living body. The physiological and/or immunological features are not particularly limited, but the MAIT-like cell can be identified by confirming expression of at least one marker that is specific to the MAIT cell. The expression of a marker can be confirmed, without being limited thereby, by known cytological methods and molecular biological methods, such as an immunological staining method using an antibody or a Reverse Transcription Polymerase-Mediated Chain Reaction (RT-PCR), and hybridization analysis. Any method for refining MAIT-like cells can be used as long as it is a known cell separation/purification method, and specific examples include flow cytometers, or magnetic beads, methods following the antigen-antibody reaction, such as the panning reaction, and cell fraction method by density gradient centrifugation using supports, such as sucrose, Percoll ("Monoclonal Antibodies: principles and practice, Third Edition" Acad. Press, 1993; "Antibody Engineering: A Practical Approach" IRL Press at Oxford University Press, 1996).

Specifically, the MAIT-like cells were positive against anti-TCR Vα7.2 antibody (3C10), anti-CD161 antibody, anti-IL-18 Rα antibody (3C10⁺/CD161⁺/IL-18Rα⁺), like MAIT cells. On the other hand, in comparison to the negative CCR7 expression of the inative MAIT cells, the MAIT-like cells that are not antigen-sensitized (immediately following creation) exhibited a weak CCR7 expression, thus, showing a difference existing between the native MAIT cells and the MAIT-like cells. Further, in comparison to MAIT cells derived from peripheral blood showing a positive expression of CD45RO, a marker of the effecter memory T cell, and a negative expression or a positive expression in just a part of the cells for CD45RA, the MAIT-like cell that is not antigen-sensitized (immediately following creation) showed almost no CD45RO positive cell, and showed a strong CD45RA expression. Accordingly, it was understood the MAIT-like cells exhibit characters quite similar to the native MAIT cells, but have a different character in some parts.

Further, MAIT-like cells established by the present invention have a cell surface antigen, a gene expression and a cytokine generating ability that are quite similar to those in the native MAIT cells, and they were confirmed as localizing and proliferating in tissues of intestines or liver when transfused in a mouse. Further, the MAIT-like cells were confirmed as having an antibacterial properties/infection resistance similar to MAIT cells. In other words, the MAIT-like cells established by the present invention were confirmed as having the same functions and features as the native MAIT cells.

One aspect of the present invention is a MAIT-like cell. The MAIT-like cell of the present invention can be used as a precious study tool to advance the functional analysis of MAIT cells. Further, the MAIT-like cells of the present invention can be used for screening to identify a new factor or substance or pharmaceutical agent that promotes the generation, differentiation induction, reproduction, survival, proliferation, etc. of MAIT cells.

The MAIT-like cell of the present invention is useful for the pharmacological assessment and activity assessment of different pharmacologically active substances, such as drugs, or new genetic products of unknown functions. For example, it can be used for substances and pharmaceutical agents relating to regulate functions of MAIT cells, and further, for screening substances and pharmaceutical agents having toxicity or being injurious to MAIT cells. Particularly, in the current situation, it is difficult to prepare sufficient MAIT cells to promote functional analysis of MAIT cells, and the features of MAIT cells are not sufficiently understood, so the MAIT-like cells prepared in the present invention will be a useful cell source to perform the above screening. Further, considering the report that most of the Tc17 cells that accumulate in the human cancer or multiple sclerosis nest (the CD8⁺ cell having an IL-17 production ability) are MAIT cells, it is beneficial to understand the function of the MAIT cell for innovative drug development.

An assay kit as disclosed herein containing MAIT-like cells prepared by the present invention is beneficial for the above screening. Further, the MAIT-like cell of the present invention can be used to create a monoclonal antibody used in the functional analysis of MAIT cells, and the screening of an agonist or antagonist that regulates the proliferation, activation and maturation of MAIT cells can be performed using MAIT-like cell of the present invention.

The subject substance to be used in screening is not particularly limited, but includes a low molecular compound, a high molecular compound, an organic compound, an inorganic compound, protein, peptide, gene, virus, cell, cell culture, and microorganism culture.

The MAIT-like cells prepared in the present invention can be provided to ex vivo coculture with lymphocytes (monocyte, dendritic cell, B cell, NK cell, T cell, etc.) prepared from the peripheral blood of a patient showing immune disorder, such as autoimmune disease, cancer, or infection, to be applied to a diagnosis of the disease, effect of the pharmaceutical agent or prediction of the prognosis of the disorder, using the change of the MAIT-like cell or the patient's lymphocyte surface antigen profile and/or the change in the generation ability of transcription factor, cytokine/chemokine, etc.

Further, the MAIT-like cells of the present invention can be used in a cell transplantation therapy by cell transplantation of the MAIT-like cells themselves or they can be administered as a cell-therapeutic agent containing the MAIT-like cells as a substantial active component. The MAIT cells enhance resistance to bacterial infection and fungal infection, so the resistance to bacterial infection or fungal infection can be improved by cell transplantation or administration of the MAIT-like cells of the present invention. Further, it is reported that MAIT cells may be involved in autoimmune disease or cancer, which means that human autoimmune disease or cancer may possibly be treated by cell transplantation of the MAIT-like cells of the present invention. In other words, there is also disclosed
a use of the MAIT-like cells in cell transplantation, MAIT-like cells for cell transplantation therapy, a therapeutic method including cell transplantation of the MAIT-like cells, and a cell-therapeutic agent containing the MAIT-like cells as a substantial active component. The patients subject to such cell transplantation therapy or cell administration therapy are patients that need to improve resistance to bacterial infection, patients receiving various cell transplantation therapies, such as organ transplantation or transplantation of hematopoietic stem cells, autoimmune disease patients, and cancer patients, but preferably, patients whose MAIT cells in blood are less than standard and/or the activity of the MAIT cells is reduced.

When performing the present invention, a person performing a method of molecular biology or genetic engineering, such as recombinant DNA technologies, and general cell biology, and the conventional art, can refer to standard books in the field, unless otherwise specified. Such books include, for example, "Molecular Cloning: A Laboratory Manual" (Sambrook & Russell, Cold Spring Harbor Laboratory Press, 3rd ed., 2001); "Current Protocols in Molecular biology" (Ausubel et al. ed., John Wiley & Sons, 1987); "Methods in Enzymology" series (Academic Press); "PCR Protocols: Methods in Molecular Biology" (Bartlett & Striling ed., Humana Press, 2003); "Animal Cell Culture: A Practical Approach" (Masters ed., Oxford University Press, 3rd ed., 2000); "Antibodies: A Laboratory Manual" (Harlow et al. & Lane ed., Cold Spring Harbor Laboratory Press, 1987).

Further, the reagents and kits for cell culture and cell biological experiments referred to in the present specification are available from commercial suppliers, such as Sigma, Invitrogen, Clontech, R&D systems or BD Bioscience.

Further, a person performing the invention can refer to standard books in the field concerning the creation, subculture, storage methods of iPS cells and other pluripotent stem cells, and common methods of cell biological experiments. Such books include, for example, "Guide to Techniques in Mouse Development" (Wasserman et al. ed., Academic Press, 1993); "Embryonic Stem Cell Differentiation in vitro" (M.V. Wiles, Meth. Enzymol. 225:900, 1993); "Manipulating the Mouse Embryo: A laboratory manual" (Hogan et al. ed., Cold Spring Harbor Laboratory Press, 1994); "Embryonic Stem Cells" (Turksen ed., Humana Press, 2002). The reagents and kits for cell culture and developmental/cell biological experiments referred to in the present specification are available from commercial suppliers, such as Invitrogen, Sigma.

### EXAMPLES

Examples are provided below to explain the present invention in more detail, but the present invention is not limited by the following Examples in any way.

### Example 1: Establishment of iPS cells from human-derived MAIT cells

Mononuclear cells were prepared from human cord blood using Ficoll. The mononuclear cells were mixed with a monoclonal antibody 3C10 (provided by Dr. Olivier Lantz of L'Institut Curie, France or Biolegend, Inc.) that had been biotin-labeled, and a MACS column (Miltenyi Biotech Inc.) using avidin magnetic beads was used to positively select cells having reactivity to the 3C10 antibody, and thus to enrich MAIT cells. This operation was performed for cord blood derived from three different donors, and the result was that MAIT cells, defined as 3C10 positive cells by the FACS analysis, had purities of 96%, 88%, 78%, respectively.

The 200,000 units of 3C10 positive cells purified as above were infected at room temperature for 2 hours with SeVdp(KOSM)302L, a vector for creating human iPS cells (provided by Dr. Mahito Nakanishi of National Institute of Advanced Industrial Science and Technology) (MOI=2.5). The vector is a Sendai virus vector loaded with the four genes derived from human (nucleic acids encoding Oct3/4, Sox2, Klf4 and c-Myc) in the same vector, and has an efficient iPS cell creating ability (WO 2010/134526). The solution containing the virus vector was removed by centrifugation, and the cells were suspended in a culture medium for ES/iPS cells composed by adding 4 ng/mL of basic fibroblast growth factor (bFGF) to a DMEM/F12 culture medium (Sigma) containing 20% Knockout Serum Replacement (KSR; Invitrogen), 0.1 mmol/L MEM non-essential amino acid solution, 2 mmol/L L-glutamine, and 0.1 mmol/L 2-mercaptoethanol, and they were seeded on a mouse embryonic fibroblast (MEF) processed by mitomycin-C, and coculture was performed at 37°C and a 5% CO₂ concentration. After 12 days, colonies exhibiting an ES/iPS-like morphology were recovered, and they were seeded on MEF seeded on 24 well plate earliers. After the colonies were cultured in a culture medium for ES/iPS cells, the grown colonies were morphologically assessed, and the colonies showing an ES/iPS-like morphology were selected.

In order to confirm whether the thus obtained iPS cells are derived from human MAIT cells, the genomic DNA of the iPS cell was used as a template to investigate whether the T cell antigen receptor α chain (TCR α) locus exhibits a gene rearrangement specific to MAIT cells. As shown in Figure 1, it is known concerning the cell having a MAIT cell-specific TCR α chain gene that the one gene encoding the TCR α region is rearranged to Vα7.2-Jα33. So, PCR was performed using the following primers by using the genomic DNA of each iPS cell clones as a template to assess whether rearrangement will occur. When there is a TCR α chain in which the Vα7.2-Jα33 is rearranged, the 282 bp band is amplified by genome PCR using the following primer, and the PCR product produces a 191+91 bp DNA fragment when digested with the restricted enzyme SacI. On the other hand, when the chain is not rearranged to Vα7.2-Jα33, the 282 bp band is not amplified.

| | |
|---|---|
| (Forward) | 5'-GGTGCCATTGTCCAGATCAACTGC-3' (SEQ ID NO: 1) |
| (Reverse) | 5'-CTTTATAATTAGCTTGGTCCCAGC-3' (SEQ ID NO: 2) |

As a result of the above confirmation, 50 or more lines of iPS cells derived from human MAIT cells (MAIT-iPS cells) were established from cells derived from three donors by performing three independent experiments.

### Example 2: Feature Analysis of MAIT-iPS cells

The MAIT-iPS cell lines established/isolated independently in Example 1, 1-3D, 2-5D, 4-6D, became single-layered flat colonies having clear contours, and showed a morphology quite similar to general human ES/iPS cells (Figure 2A).

Further, the MAIT-iPS cells whose subculture was maintained as above were studied concerning expression of markers specific to human ES/iPS cells. The fixed MAIT-iPS cells were reacted with a primary antibody selected from an anti-alkaline phosphatase (ALP) antibody, an anti- SSEA4 antibody, an anti-Oct-3/4 antibody, an anti-Nanog antibody (all from R&D System), an anti-TRA-1-60 antibody (BD Bioscience) or an anti-TRA-1-81 antibody (Santa Cruz), then they were stained using a Rhodamine labeled secondary antibody (Jackson ImmunoResearch). The cell nucleus was stained with a 4',6-diamidino-2-phenylindole (DAPI) solution (1 µg/mL). The staining images of these antibodies and coloring matters were observed under a fluorescence microscope. As a result, the MAIT-iPS cell was strongly positive for all of alkaline phosphatase, SSEA4, Oct-3/4, Nanog, TRA-1-60 and TRA-1-81 (Figure 2B).

Similarly, an RNA of the MAIT-iPS cell was prepared, and the expression of genes specific to undifferentiated human iPS cells, Oct-3/4 and Nanog, was confirmed. The total RNA prepared from a MAIT-iPS cell or a human iPS cell were used to synthesize cDNA, and the resulted cDNAs was used as a template to perform a polymerase chain reaction (PCR) using the following primers, to amplify many gene fragments.
Oct-3/4 [Amplification size: 144 bp]

| | |
|---|---|
| (Forward) | 5'- GACAGGGGGAGGGGAGGAGCTAGG-3' (SEQ ID NO: 3) |
| (Reverse) | 5'- CTTCCCTCCAACCAGTTGCCCCAAAC-3' (SEQ ID NO: 4) |

Nanog [Amplification size: 391 bp]

| | |
|---|---|
| (Forward) | 5'- CAGCCCCGATTCTTCCACCAGTCCC-3' (SEQ ID NO: 5) |
| (Reverse) | 5'- CGGAAGATTCCCAGTCGGGTTCACC-3' (SEQ ID NO: 6) |

GAPDH (glyceraldehyde-3-phosphate dehydrogenase) [Amplification size: 382 bp]

| | |
|---|---|
| (Forward) | 5'-AATCCCATCACCATCTTCC-3' (SEQ ID NO: 7) |
| (Reverse) | 5'- CATCACGCCACAGTTTCC-3' (SEQ ID NO: 8) |

The PCR product was subjected to electrophoresis with 1.5% agarose gel, and stained with ethidium bromide (Merck & Co.), then detected using a gel documentation system (ATTO). Strong expression of Oct-3/4 and Nanog genes were observed similar to human iPS cells in different cell lines of 1-3D, 2-5D, 4-6D, A11, A13, C4B, C5B, which are individually established/isolated MAIT-iPS cells (Figure 3).

In order to study the gene expression of the MAIT-iPS cells more exhaustively, the DNA microarray (Agilent Technologies) analysis was performed, and the correlation of gene expression profiles in the MAIT-iPS cell and the human iPS/ES cell were studied. The result is shown in Table 1. As shown in Table 1, the MAIT-iPS cell shows a gene expression pattern quite similar to a human ES cell or a human iPS cell (the correlation coefficient was 0.95 or higher in all comparisons). On the other hand, the result of the MAIT-iPS cell differed from that of the MAIT cell derived from human cord blood, which is the creation source cell of the MAIT-iPS cell. In addition, the MAIT-iPS cell is confirmed as exhibiting demethylation of an Oct-3/4 gene or a Nanog gene promoter region, and a high telomerase activity, similar to a general human ES/iPS cell, and it allows cultivation of a subculture while maintaining the undifferentiated character.

The above result shows that iPS cell lines established from MAIT cells (MAIT-iPS cell lines) have a general iPS cell-like feature/function.

### [Table 1]

**Table 1 Comparison of Gene Expression Form of Different MAIT-IPS cell lines and human ES cells, human iPS cells**

| | | MAIT | C5B | C4B | A13 | A11 | 4-6D | 2-5D | 1-3D | iPS | ES |
|---|---|---|---|---|---|---|---|---|---|---|---|
| ES | | 0.805 | 0.969 | 0.971 | 0.968 | 0.966 | 0.970 | 0.969 | 0.974 | 0.972 | 1.000 |
| iPS | | 0.800 | 0.965 | 0.965 | 0.964 | 0.975 | 0.967 | 0.967 | 0.967 | 1.000 | |
| MAIT-iPS | 1-3D | 0.807 | 0.985 | 0.985 | 0.982 | 0.978 | 0.987 | 0.986 | 1.000 | | |
| | 2-5D | 0.807 | 0.987 | 0.983 | 0.985 | 0.980 | 0.986 | 1.000 | | | |
| | 4-6D | 0.810 | 0.986 | 0.987 | 0.984 | 0.979 | 1.000 | | | | |
| | A11 | 0.806 | 0.983 | 0.978 | 0.981 | 1.000 | | | | | |
| | A13 | 0.808 | 0.986 | 0.983 | 1.000 | | | | | | |
| | C4B | 0.807 | 0.987 | 1.000 | | | | | | | |
| | C5B | 0.805 | 1.000 | | | | | | | | |
| MAIT | | 1.000 | | | | | | | | | |

| | | | | | | | | | | | |
|---|---|---|---|---|---|---|---|---|---|---|---|
| DNA array analysis of different MAIT-IPS cell lines and human ES cells, human iPS cells was performed to compare the gene expression form of the cells. The numbers show the correlation coefficient between the cells. ES: Human ES cells (line khE3), iPS: human iPS cell (line B7), MAIT: human cord blood-derived MAIT cells | | | | | | | | | | | |

Note that in the following experiment, a plurality of cell lines, such as the 1-3D line, 2-5D line and 4-6D line, were used as the MAIT-iPS cell, like shown above, but no c change in experimental results due to difference of used cell lines was seen in general. Accordingly, the Example data of 1-3D line is used unless otherwise specified in the following Examples.

The differentiation ability of the MAIT-iPS cell was further studied. The MAIT-iPS cells cultured on MEF while maintaining the undifferentiated character was treated with a cell dissociation solution (including 0.25% trypsin, 1 mg/ml collagenase IV) to form a small mass, and suspended in a culture medium for ES/iPS cells that do not include bFGF, then seeded on a low adhesive plate. Cell aggregated masses were recovered 8 days later, then they were seeded on a cell adhesive plate pretreated with gelatin to be fixed 16 days later. The fixed cells were reacted with primary antibodies, such as an anti-muscle actin antibody (Nichirei Bioscience) or anti-Sox-17 antibody (R&D System), anti-nestin antibody (Sigma), then stained and observed by fluorescence microscope as shown above. Consequently, it was confirmed that a muscle actin positive mesoderm cell, a Sox-17 positive endoderm cell, a nestin positive ectoderm cell appeared.

Further, 8×10⁶ to 10×10⁶ MAIT-iPS cells were transplanted subcutaneously to a NOD/scid mouse (Charles River), and 10 to 14 weeks later, teratoma formation was observed. Tissue fragments were created from paraffin embedded samples of tumors, and reacted with a primary antibody, which is an anti-pan cytokeratin antibody (DAKO) or an anti-desmin antibody (DAKO), and then reacted with a biotin-labeled secondary antibody (DAKO), and finally subjected to a coloring reaction using diaminobenzidine. When the subject was observed by optical microscope after it was stained with a hematoxylin solution, a neural tube-like structure including a melanin positive cell, an intestine-like structure composed of a cytokeratin positive cell, and further a desmin positive muscle cell-like tissue were found to exist (Figure 4).

Based on the above result, it was confirmed that MAIT-iPS cells express the marker genes and proteins specific to an undifferentiated state, similar to general human ES/iPS cells, and have pluripotency to differentiate to all three lineages of cells.

### Example 3: Creating MAIT cells from MAIT-iPS cells

It is known that it is possible to induce differentiation of stem cells, such as ES cells, to CD34 positive precursor cells of hemocyte/lymphocyte by coculture with OP9 cell as a feeder cell, and it is al so possible to induce differentiation of such precursor cells to Tcell linage cells by coculture with DLL1-expressed OP9 cells (OP9/DLL1) wherein the DLL1 is one of Notch ligands (Schmitt TM et al., Nat. Immun. 5, 410-417 (2004); Wakao H et al., FASEB J. 22, 2223-2231 (2008); Wakao H et al., WO2008/038579; Watarai H et al., J. Clin. Invest. 120, 2610-2618 (2010); WO 2010/027094; Timmermans F et al., J. Immunol. 182, 6879-6888 (2011)).

In the present Examples, an attempt to induce differentiation from MAIT-iPS cells to MAIT cells was made based on said method.

OP9 cells and OP9/DLL1 cells obtained from the Riken BioResource Center (Riken Cell Bank) were used. First, dispersed small masses of about 100 colonies of the MAIT-iPS cells were seeded at 1×10⁶ per a 10 cm dish on OP9 cells made confluent in the dishes 3 to 7 days earlier, cultured in an α MEM culture medium containing a 10% fetal bovine serum (FBS) and 0.1 mM 1-thioglycerol, and were differentiated to stem cells/precursor cells of hemocytes and lymphocytes. Cell aggregates formed in a colony-like state 11 to 12 days after seeding were washed twice with a phosphate buffered solution (PBS), then an α MEM culture medium containing 1 mg/mL of collagenase IV (Invitrogen) and 0.01% trypsin/EDTA (Sigma) was added to those aggregates and agitated well to disperse the aggregates to single cells. From these cell groups, CD34 positive cell fractions (purity of 95% or higher) were formed using a CD34 MultiSort Kit (Miltenyi) and recovered, then suspended in an α MEM culture medium containing a 20% FBS, a human SCF (stem cell factor), human interleukin (IL-7), human Flt3 ligand (FL) (all from Reprotech, 5 ng/mL each) (referred to hereinafter as MAIT cell-differentiation inducing culture medium), then seeded in 24 well cell culture plate on OP9/DLL1 cells made confluent on the plate 3 to 7 days earlier. Half of the MAIT cell-differentiation inducing culture medium was changed every 4 days, while cultivation was continued for 14 to 30 days, then the cells were recovered by separating them from feeder cells by pipet operation.

Accordingly, the character of the cells differentiated from the MAIT-iPS cells was considered by flow cytometry (FCM) using reactivity to anti-TCR Vα 7.2 antibody (3C10), anti-TCR αβ antibody (IP26; Biolegend), anti-CD161 antibody (DX12; BD Bioscience) and anti-IL-18Rα antibody (H44; Biolegend) as the index. The result is shown in Figure 5.

Firstly, when the differentiated cells derived from the MAIT-iPS cells prepared by the above method (30 days from seeding to the OP9/DLL1 cells) were stained with the above four types of antibodies, most of those cells turned to TCR Vα7.2⁺/TCRαβ⁺ (the TCR Vα7.2⁺ will be referred to as 3C10⁺ (3C10 antigen positive) hereinafter). Secondly, the double-positive fraction cell is at the same time mostly CD161⁺ and IL-18Rα⁺, showing that MAIT-like cells can be differentiated from MAIT-iPS cells.

The MAIT-like cells can be created with good reproducibility and high efficiency by the present method, and the MAIT-like cells defined by 3C10⁺/TCRαβ⁺/CD161⁺/IL-18Rα⁺ occupied 85% or higher to the total for all of the multiple attempts.

The MAIT-like cells obtained from induced differentiation of the MAIT-iPS cells using the present method are referred to as iMAIT cells.

### Example 4: Property Analysis of iMAIT cells

From a series of reports by Lantz et al. (Dusseaux et al., 2011), the MAIT cells existing in vivo, particularly in the peripheral blood, are known to show positive in cell surface markers, such as CD26 (DPP-IV), CD27, CD28, CD62L (L-selectin), CD95(Fas), CD127 (IL-7Rα), CD244 (SLAMF4) as well as TCR Vα7.2, CD161, IL-18Rα. Accordingly, it was investigated what the reactivity of the iMAIT cells to antibodies specific to these markers is.

As a positive control, 3C10⁺/TCRαβ⁺/ CD161⁺ fraction cells were prepared from the human peripheral blood mononuclear cells (Cellular Technology), similarly to the above method, and said cells were used as human peripheral blood-derived MAIT cells (hereinafter referred to as PBMC-MAIT cells).

### [Table 2]

**Table 2 Expression of Different Surface Antigen Marker in PBMC-MAIT and iMAIT Cells.**

| | PBMC-MAIT | | iMAIT |
|---|---|---|---|
| | Published Article | Analysis Result | |
| 3C10 | + | + | + |
| TCR*α β* | + | + | + |
| CD3 | + | + | + |
| CD161 | H | H | H |
| IL-18R*α* | + | + | + |
| CD26 | H | H | H |
| CD27 | H | +∼H | L |
| CD28 | H | +∼H | L |
| CD62L | L | -∼L | L |
| CD95 | H | + | + |
| CD127 | H | +∼H | L |
| CD244 | H | +∼H | H |
| CCR5 | H | + | + |
| CCR6 | H | + | H |
| CCR7 | - | - | L |
| CD45RO | + | + | - |
| CD45RA | - (or L) | - (or L) | + |

| | | | |
|---|---|---|---|
| Intensity of the expression of different surface antigen markers are shown as positive (+), negative (-). The most positive and weakly positive expression are respectively shown as H, L. | | | |

The result is shown in Table 2. As already reported, expressions of CD26, CD27, CD28, CD62L, CD95, CD127, CD244 were seen for the PBMC-MAIT cell. All these markers were expressed in the iMAIT cell as well, although there were some differences in intensity of the expression (Figure 6, Table 2).

Further, the PBMC-MAIT cell is known to strongly express chemokine receptors, such as CCR5 or CCR6, but its CCR7 expression is negative (Dusseaux et al., 2011). When the chemokine receptor expression in the PBMC-MAIT cell and the iMAIT cell is studied by a method similar to the above, the iMAIT cell showed strong CCR5 and CCR6 expression like the PBMC-MAIT cell. Meanwhile, a weak expression of CCR7 was confirmed, showing that the iMAIT cell has a different feature from PBMC-MAIT cell (Table 2).

Further, the PBMC-MAIT cell generally exhibits a positive expression of CD45RO, which is a marker of an effecter memory T cell, and the expression of CD45RA known as a marker for the naïve T cell (T cell before receiving antigen stimulus) is negative, and positive for only part of the cells, but in the iMAIT cell, a strong CD45RA expression was seen and almost no CD45RO positive cell was seen (Figure 6, Table 2).

As shown from the above result, the iMAIT cell strongly showed having a similar character to the PBMC-MAIT cell, but its character differs in part from the PBMC-MAIT, since it maintains the feature of a naive T cell defined by expression of CD45RA or CCR7.

### Example 5: Confirmation of Cytokine Production Ability of iMAIT cells

A known feature of MAIT cells is that a costimulus signal against CD3/TCR and CD28 (referred to hereinafter as CD3/CD28 stimulus), or a stimulus by phorbol 12-myristate 13-acetate (PMA) and Ionomycin (hereinafter referred to PMA/Iono stimulus) induce to produce cytokines, such as interferon (IFN) γ (Dusseaux et al., 2011). Hence, the iMAIT cells produced based on the method of Examples 2, 3 were mixed with magnetic beads (Dynabeads Human T-Activator CD3/CD28; Invitrogen) coated with anti-CD3 antibody and anti-CD28 antibody and cultured under 48 hours of CD3/CD28 stimulus. Further, PMA (10 ng/mL; Wako Pure Chemicals Industry, Ltd.) and Ionomycin (1 µM; Wako Pure Chemicals Industry, Ltd.) were added to the iMAIT cell culture and a PMA/Iono stimulus was performed for 48 hours.

Then, the culture supernatant was recovered and the amounts of different cytokines were measured using a Bioplex Pro-Human Cytokine 21-plex Assay and Pro-Human Cytokine 27-plex Assay system (BioRad). As a result, it was found that the iMAIT cell does not produce IFNγ at an unstimulated state, but a significantly high IFNγ production was seen when a CD3/CD28 stimulus or a PMA/Iono stimulus was added (Figure 7). An investigation of other cytokine production abilities revealed that the iMAIT cell does not produce IL-2, IL-17, TNF-α at an unstimulated state, but exhibited a clear production enhancing effect by PMA/Iono stimulus. On the other hand, there was no production of IL-4 or IL-10, and these results indicate a tendency similar to PBMC-MAIT cells.

Further, the MAIT cell is known to produce cytokines, such as IFNγ, by coculture with a monocyte-fed bacteria. So, the cytokine production ability of an iMAIT cell was studied using the same system. The mononuclear cells derived from human peripheral blood was seeded 1×10⁵ cells each to a 96 well cell culture plate, then left for an hour, after which the cells left on the bottom surface as adhesive cells (about 1×10⁴ cells) were used as monocytes. The adhesive cells in the serum-free/additive-free DMEM culture medium was infected for 3 hours with Escherichia coli (multiplicity of infection: MOI=100). Then, it was washed and replaced with DMEM to which 10% FBS and penicillin-streptomycin were added, and 2×10⁴ iMAIT cells, prepared by a method similar to Examples 2, 4, were seeded per plate. After 48 hours of coculture, the supernatant of the culture was recovered, and the amount of different cytokines was measured. As a result, an increase in the production amount of cytokine, such as IFNγ, IL-2, IL-17, TNF-α, was observed also in said coculture system.

### Example 6: Study of the colonization of iMAIT cells in the mouse body and their infection prevention effect

To confirm whether the iMAIT cells behave similarly to normal MAIT cells, iMAIT cells (5×10⁴ cells/mouse) produced in Example 3 were infusion grafted by transvenous administration to a NOD/scid mouse of 8 to 10 weeks old irradiated with radiation of 320 cGy before hand (Charles River). The mouse was euthanized 6 to 10 weeks later, then the bone marrow, liver, spleen, small intestine epithelium, or proper mucous membrane were dissected. Then, lymphocytes were recovered from the organs, and the existence of iMAIT cells, that is, 3C10⁺/TCRαβ⁺ cells were studied using the same FCM method as Example 3.

The result was that the iMAIT cells existed in all organs examined, and it was accumulated at an especially high ratio in the lamina propria (Figure 8). Further, an example was found in which the number of iMAIT cells detected in the lamina propria is at least 100 folds the number of injected cells, indicating that the iMAIT cells are proliferating in the mouse in vivo environment.

The MAIT cell is known to react with cells infected with bacteria, such as tubercle bacillus, and to have an infection prevention function. The iMAIT cell produces not only IFNγ, IL-17, or IL-2 that plays an important role in preventing infection (Example 5), but also perforin and granzyme that play a central role in inducing cytotoxicity, similarly to the cytokines (data not indicated). Hence, whether iMAIT cells actually show a protective effect against bacteria infection was considered.

The iMAIT cells (5×10⁴ cells/mouse) created by the method of Example 3 was infusion grafted by transvenous administration to a NOG (NOD.Cg-Prkdc scid Il2rgtm1Sug/Jic) mouse (Central Institute for Experimental Animals). Mycobacterium abscessus was inoculated at 1.0×10⁶ CFU/mouse 5 weeks later. The mouse was euthanized 2 weeks later, and the liver and spleen were dissected to investigate the number of living M. abscessus in the organs (colony production ability).

As seen from the result in Figure 9, the group iMAIT(+), to which iMAIT cells are transplanted, shows a significant reduction in the viable cell (formed colony) count for liver, and a same tendency was witnessed in the spleen.

The above experiment result clearly shows that the iMAIT cells will exhibit function or effect that is equivalent to MAIT cells.

## Claims

1. A preparation method of a MAIT-like cell comprising:
introducing a reprogramming factor to a MAIT cell to obtain an induced pluripotent stem cell retaining a TCR α chain gene rearranged to a MAIT cell-specific fashion then
inducing differentiation of the induced pluripotent stem cell to obtain a MAIT-like cell.

2. A preparation method of an induced pluripotent stem cell comprising:
introducing a reprogramming factor to a MAIT cell to obtain an induced pluripotent stem cell retaining a TCR α chain gene rearranged to a MAIT cell-specific fashion.

3. The method according to either Claim 1 or 2, wherein the reprogramming factor is introduced using a virus vector.

4. The method according to Claim 3, wherein the virus vector is Sendai virus vector.

5. The method according to Claim 4, wherein the Sendai virus vector incorporates a plurality of reprogramming factors in a same vector.

6. An induced pluripotent stem cell retaining a TCR α chain gene rearranged to a MAIT cell-specific fashion.

7. The induced pluripotent stem cell according to Claim 6 retaining only a single TCR α chain gene rearranged to a MAIT cell-specific fashion as a TCR α chain gene.

8. The induced pluripotent stem cell according to either Claim 6 or 7, wherein the TCR α chain gene rearranged to a MAIT cell-specific fashion is Vα 7.2-Jα 33 for a human and Vα 19-Jα 33 for a mouse.

9. The induced pluripotent stem cell according to any one of Claims 6 to 8 obtained by any method of Claims 2 to 5.

10. A preparation method of a MAIT-like cell comprising:
inducing differentiation of the induced pluripotent stem cell according to any one of Claims 6 to 9 to obtain a MAIT-like cell.

11. The method according to Claim 10 comprising coculture the induced pluripotent stem cell with a feeder cell to obtain a MAIT-like cell.

12. A MAIT-like cell obtained by the method according to either Claim 10 or 11.

13. The MAIT-like cell according to Claim 12 exhibiting a positive expression of CD45RA.

14. An evaluation method of an activity to regulate a MAIT cell function of a test substance comprising a step of bringing the MAIT-like cell according to Claim 12 or 13 in contact with the test substance.

15. A cell therapy agent containing the MAIT-like cell according to Claims 12 or 13.

16. The cell therapy agent according to Claim 15 that is administered to improve resistance against bacterial infection or fungal infection.

## Patentansprüche

1. Herstellungsverfahren einer MAIT-ähnlichen (MAIT-like) Zelle, umfassend:
Einführen eines Reprogrammierungsfaktors in eine MAIT-Zelle, um eine induzierte pluripotente Stammzelle zu erhalten, die ein TCR-α-Kette-Gen beibehält, das in MAIT-zellspezifischer Weise umgelagert (rearranged) ist, und
anschließende Induktion von Differenzierung der induzierten pluripotenten Stammzelle, um eine MAIT-ähnliche Zelle zu erhalten.

2. Herstellungsverfahren einer induzierten pluripotenten Stammzelle, umfassend:
Einführen eines Reprogrammierungsfaktors in eine MAIT-Zelle, um eine induzierte pluripotente Stammzelle zu erhalten, die ein TCR-α-Kette-Gen beibehält, das in MAIT-zellspezifischer Weise umgelagert ist.

3. Verfahren nach Anspruch 1 oder 2, wobei der Reprogrammierungsfaktor durch Verwendung eines Virus-Vektors eingeführt wird.

4. Verfahren nach Anspruch 3, wobei der Virus-Vektor ein Sendai-Virus-Vektor ist.

5. Verfahren nach Anspruch 4, wobei der Sendai-Virus-Vektor eine Vielzahl von Reprogrammierungsfaktoren in demselben Vektor einschließt.

6. Induzierte pluripotente Stammzelle, die ein TCR-α-Kette-Gen beibehält, das in MAIT-zellspezifischer Weise umgelagert ist.

7. Induzierte pluripotente Stammzelle nach Anspruch 6, die nur ein einzelnes TCR-α-Kette-Gen, das in MAIT-zellspezifischer Weise umgelagert ist, als TCR-α-Kette-Gen beibehält.

8. Induzierte pluripotente Stammzelle nach Anspruch 6 oder 7, wobei das TCR-α-Kette-Gen, das in MAIT-zellspezifischer Weise umgelagert ist, Vα 7.2-Ja 33 für einen Menschen und Vα 19-Jα 33 für eine Maus ist.

9. Induzierte pluripotente Stammzelle nach einem der Ansprüche 6 bis 8, die durch ein Verfahren nach den Ansprüchen 2 bis 5 erhalten wurde.

10. Herstellungsverfahren einer MAIT-ähnlichen Zelle, umfassend:
Induzieren der Differenzierung der induzierten pluripotenten Stammzelle nach einem der Ansprüche 6 bis 9, um eine MAIT-ähnliche Zelle zu erhalten.

11. Verfahren nach Anspruch 10, umfassend das Co-Züchten der induzierten pluripotenten Stammzelle mit einer Feederzelle, um eine MAIT-ähnliche Zelle zu erhalten.

12. MAIT-ähnliche Zelle, die durch das Verfahren nach Anspruch 10 oder 11 erhalten wurde.

13. MAIT-ähnliche Zelle nach Anspruch 12, die eine positive Expression von CD45RA aufweist.

14. Bewertungsverfahren einer Aktivität, um eine MAIT-Zellfunktion von einer Testsubstanz zu regulieren, umfassend einen Schritt des Inkontaktbringens der MAIT-ähnlichen Zelle nach Anspruch 12 oder 13 mit der Testsubstanz.

15. Zelltherapie-Agens, das die MAIT-ähnliche Zelle nach Anspruch 12 oder 13 enthält.

16. Zelltherapie-Agens nach Anspruch 15, das verabreicht wird, um die Resistenz gegen eine bakterielle Infektion oder eine Pilzinfektion zu verbessern.

## Revendications

1. Procédé de préparation d'une cellule de type MAIT comprenant :
l'introduction d'un facteur de reprogrammation dans une cellule MAIT afin d'obtenir une cellule souche pluripotente induite conservant un gène de chaîne α de TCR réarrangé de manière spécifique à une cellule MAIT, puis
l'induction de la différenciation de la cellule souche pluripotente induite afin d'obtenir une cellule de type MAIT.

2. Procédé de préparation d'une cellule souche pluripotente induite comprenant :
l'introduction d'un facteur de reprogrammation dans une cellule MAIT afin d'obtenir une cellule souche pluripotente induite conservant un gène de chaîne α de TCR réarrangé de manière spécifique à une cellule MAIT.

3. Procédé selon la revendication 1 ou 2, dans lequel le facteur de reprogrammation est introduit en utilisant un vecteur viral.

4. Procédé selon la revendication 3, dans lequel le vecteur viral est un vecteur basé sur le virus Sendai.

5. Procédé selon la revendication 4, dans lequel le vecteur basé sur le virus Sendai incorpore une pluralité de facteurs de reprogrammation dans un même vecteur.

6. Cellule souche pluripotente induite conservant un gène de chaîne α de TCR réarrangé de manière spécifique à une cellule MAIT.

7. Cellule souche pluripotente induite selon la revendication 6, conservant uniquement un seul gène de chaîne α de TCR réarrangé de manière spécifique à une cellule MAIT en tant que gène de chaîne α de TCR.

8. Cellule souche pluripotente induite selon la revendication 6 ou 7, dans laquelle le gène de chaîne α de TCR réarrangé de manière spécifique à une cellule MAIT est Vα 7.2-Jα 33 pour un humain et Vα 19-Jα 33 pour une souris.

9. Cellule souche pluripotente induite selon l'une quelconque des revendications 6 à 8 obtenue par un quelconque procédé selon les revendications 2 à 5.

10. Procédé de préparation d'une cellule de type MAIT comprenant :
l'induction de la différenciation de la cellule souche pluripotente induite selon l'une quelconque des revendications 6 à 9 afin d'obtenir une cellule de type MAIT.

11. Procédé selon la revendication 10, comprenant la co-culture de la cellule souche pluripotente induite avec une cellule nourricière afin d'obtenir une cellule de type MAIT.

12. Cellule de type MAIT obtenue par le procédé selon la revendication 10 ou 11.

13. Cellule de type MAIT selon la revendication 12, exhibant une expression positive de CD45RA.

14. Procédé d'évaluation de l'activité d'une substance de test à réguler la fonction d'une cellule MAIT, comprenant une étape consistant à mettre en contact la cellule de type MAIT selon la revendication 12 ou 13 avec la substance de test.

15. Agent de thérapie cellulaire contenant la cellule de type MAIT selon les revendications 12 ou 13.

16. Agent de thérapie cellulaire selon la revendication 15, qui est administré pour améliorer une résistance contre une infection bactérienne ou une infection fongique.


### SEQUENCE LISTING

<110> DAIICHI SANKYO COMPANY, LIMITED and
   NATIONAL UNIVERSITY CORPORATION HOKKAIDO UNIVERSITY
<120> MAIT-LIKE CELLS AND PROCESS FOR PRODUCING THE SAME
<130> FA1511-13114
<150> JP 2012-239195
   <151> 2012-10-30
<160> 14
<170> Patent In version 3.5
<210> 1
   <211> 24
   <212> DNA
   <213> Artificial Sequence
<220>
   <223> Forward Primer for MAIT-TCR gene
<400> 1
   ggtgccattg tccagatcaa ctgc 24
<210> 2
   <211> 24
   <212> DNA
   <213> Artificial Sequence
<220>
   <223> Reverse Primer for MAIT-TCR gene
<400> 2
   ctttataatt agcttggtcc cagc 24
<210> 3
   <211> 21
   <212> DNA
   <213> Artificial Sequence
<220>
   <223> Forward Primer for Oct3/4 cDNA
<400> 3
   gaagttggag aaggtggaac c 21
<210> 4
   <211> 21
   <212> DNA
   <213> Artificial Sequence
<220>
   <223> Reverse Primer for Oct3/4 cDNA
<400> 4
   gcctcatact cttctcgttg g 21
<210> 5
   <211> 20
   <212> DNA
   <213> Artificial Sequence
<220>
   <223> Forward Primer for Nanog cDNA
<400> 5
   aaagtgagat tagccccgag 20
<210> 6
   <211> 20
   <212> DNA
   <213> Artificial Sequence
<220>
   <223> Reverse Primer for Nanog cDNA
<400> 6
   tcccatcccc ttcaatagca 20
<210> 7
   <211> 19
   <212> DNA
   <213> Artificial Sequence
<220>
   <223> Forward Primer for GAPDH cDNA
<400> 7
   ggaagcttgt catcaacgg 19
<210> 8
   <211> 20
   <212> DNA
   <213> Artificial Sequence
<220>
   <223> Reverse Primer for GAPDH cDNA
<400> 8
   ctcttgctca gtgtccttgc 20
<210> 9
   <211> 1599
   <212> DNA
   <213> Homo sapiens
<400> 9
   aacatttcca aatcttggca ttcttatcca caaagtgaag ataataattg tcaattcaca 60
   ggtgattatg atttaaagag attacttttg aagagttcct aacacattca gtcaacattt 120
   aatgatgctt caggcactgt gttcattgct agtgagcgta tgacacacac agccatacgg 180
   tcacagagct ttcaatgaaa agtaacataa ttgctcattt caccaggccc ccggcttggg 240
   gcgccttcct tccccatggc gggacacctg gcttcggatt tcgccttctc gccccctcca     300
   ggcggtgggg gtgatgggcc atggggggcg gagccgggct gggttgatcc tctgacctgg     360
   ctaagcttcc aaggccctcc tggagggcca ggaatcgggc cgggggttgg gccaggctct     420
   gaggtgtggg ggattccccc ttgccccccg ccgtatgagt tatgtggggg gatggcgtac      480
   tgtgggcctc aggttggagt ggggctagtg ccccaaggcg gcttggagac ctctcagcct     540
   gagagcgaag caggagtcgg ggtggagagc aactccaatg gggcctcccc ggaaccctgc     600
   accgtccccc ctggtgccgt gaagctggag aaggagaagc tagagcaaaa cccggagaag     660
   tcccaggaca tcaaagctct gcagaaagaa ctcgagcaat ttgccaagct cctgaagcag     720
   aagaggatca ccctgggata tacacaggcc gatgtggggc tcatcctggg ggttctattt     780
   gggaaggtgt tcagccaaaa gaccatctgc cgctttgagg ctctgcagct tagcttcaag     840
   aacatgtgta agctgcggcc cttgctgcag aagtgggtgg aggaagctga caacaatgaa     900
   aatcttcagg agatatgcaa agcagaaacc ctcatgcagg cccgaaagag aaagcgaacc     960
   agtatcgaga accgagtgag aggcaacctg gagaatttgt tcctgcagtg cccgaaaccc    1020
   acactgcaga tcagccacat cgcccagcag cttgggctcg agaaggatgt ggtccgagtg    1080
   tggttctgta accggcgcca gaagggcaag cgatcaagca gcgactatgc acaacgagag    1140
   gattttgagg ctgctgggtc tcctttctca gggggaccag tgtcctttcc tccggcccca    1200
   gggccccatt ttggtacccc aggctatggg agccctcact tcactgcact gtactcctca    1260
   gtccctttcc ctgaggggga agtctttccc ccagtctccg tcatcactct gggctctccc   1320
   atgcattcaa actgaggtgc ctgcccttct aggaatgggg aacaggggag gggaggagct    1380
   agggaaagag aacctggagt ttgtggcagg gcttttggga ttaagttctt cattcactaa    1440
   ggaaggaatt gggaacacta agggtggggg caggggagtt tggggcaact ggttggaggg    1500
   aaggtgaagt tcaatgatgc tcttgatttt aatcccacat catgtatcac ttttttctta   1560
   aataaagaag cctgggacac agtaaaaaaa aaaaaaaaa                           1599
<210> 10
   <211> 2949
   <212> DNA
   <213> Homo sapiens
<400> 10
   agtttcccga ccagagagaa cgaacgtgtc tgcgggcgcg cggggagcag aggcggtggc      60
   gggcggcggc ggcaccggga gccgccgagt gaccctcccc cgcccctctg gccccccacc     120
   ctcccacccg cccgtggccc gcgcccatgg ccgcgcgcgc tccacacaac tcaccggagt     180
   ccgcgccttg cgccgccgac cagttcgcag ctccgcgcca cggcagccag tctcacctgg     240
   cggcaccgcc cgcccaccgc cccggccaca gcccctgcgc ccacggcagc actcgaggcg     300
   accgcgacag tggtggggga cgctgctgag tggaagagag cgcagcccgg ccaccggacc 360
   tacttactcg ccttgctgat tgtctatttt tgcgtttaca acttttctaa gaacttttgt 420
   atacaaagga actttttaaa aaagacgctt ccaagttata tttaatccaa agaagaagga 480
   tctcggccaa tttggggttt tgggttttgg cttcgtttct tctcttcgtt gactttgggg 540
   ttcaggtgcc ccagctgctt cgggctgccg aggaccttct gggcccccac attaatgagg 600
   cagccacctg gcgagtctga catggctgtc agcgacgcgc tgctcccatc tttctccacg 660
   ttcgcgtctg gcccggcggg aagggagaag acactgcgtc aagcaggtgc cccgaataac 720
   cgctggcggg aggagctctc ccacatgaag cgacttcccc cagtgcttcc cggccgcccc 780
   tatgacctgg cggcggcgac cgtggccaca gacctggaga gcggcggagc cggtgcggct 840
   tgcggcggta gcaacctggc gcccctacct cggagagaga ccgaggagtt caacgatctc 900
   ctggacctgg actttattct ctccaattcg ctgacccatc ctccggagtc agtggccgcc 960
   accgtgtcct cgtcagcgtc agcctcctct tcgtcgtcgc cgtcgagcag cggccctgcc 1020
   agcgcgccct ccacctgcag cttcacctat ccgatccggg ccgggaacga cccgggcgtg 1080
   gcgccgggcg gcacgggcgg aggcctcctc tatggcaggg agtccgctcc ccctccgacg 1140
   gctcccttca acctggcgga catcaacgac gtgagcccct cgggcggctt cgtggccgag 1200
   ctcctgcggc cagaattgga cccggtgtac attccgccgc agcagccgca gccgccaggt 1260
   ggcgggctga tgggcaagtt cgtgctgaag gcgtcgctga gcgcccctgg cagcgagtac 1320
   ggcagcccgt cggtcatcag cgtcagcaaa ggcagccctg acggcagcca cccggtggtg 1380
   gtggcgccct acaacggcgg gccgccgcgc acgtgcccca agatcaagca ggaggcggtc 1440
   tcttcgtgca cccacttggg cgctggaccc cctctcagca atggccaccg gccggctgca 1500
   cacgacttcc ccctggggcg gcagctcccc agcaggacta ccccgaccct gggtcttgag 1560
   gaagtgctga gcagcaggga ctgtcaccct gccctgccgc ttcctcccgg cttccatccc 1620
   cacccggggc ccaattaccc atccttcctg cccgatcaga tgcagccgca agtcccgccg 1680
   ctccattacc aagagctcat gccacccggt tcctgcatgc cagaggagcc caagccaaag 1740
   aggggaagac gatcgtggcc ccggaaaagg accgccaccc acacttgtga ttacgcgggc 1800
   tgcggcaaaa cctacacaaa gagttcccat ctcaaggcac acctgcgaac ccacacaggt 1860
   gagaaacctt accactgtga ctgggacggc tgtggatgga aattcgcccg ctcagatgaa 1920
   ctgaccaggc actaccgtaa acacacgggg caccgcccgt tccagtgcca aaaatgcgac 1980
   cgagcatttt ccaggtcgga ccacctcgcc ttacacatga agaggcattt ttaaatccca 2040
   gacagtggat atgacccaca ctgccagaag agaattcagt attttttact tttcacactg 2100
   tcttcccgat gagggaagga gcccagccag aaagcactac aatcatggtc aagttcccaa 2160
   ctgagtcatc ttgtgagtgg ataatcagga aaaatgagga atccaaaaga caaaaatcaa 2220
   agaacagatg gggtctgtga ctggatcttc tatcattcca attctaaatc cgacttgaat 2280
   attcctggac ttacaaaatg ccaagggggt gactggaagt tgtggatatc agggtataaa 2340
   ttatatccgt gagttggggg agggaagacc agaattccct tgaattgtgt attgatgcaa 2400
   tataagcata aaagatcacc ttgtattctc tttaccttct aaaagccatt attatgatgt 2460
   tagaagaaga ggaagaaatt caggtacaga aaacatgttt aaatagccta aatgatggtg 2520
   cttggtgagt cttggttcta aaggtaccaa acaaggaagc caaagttttc aaactgctgc 2580
   atactttgac aaggaaaatc tatatttgtc ttccgatcaa catttatgac ctaagtcagg 2640
   taatatacct ggtttacttc tttagcattt ttatgcagac agtctgttat gcactgtggt 2700
   ttcagatgtg caataatttg tacaatggtt tattcccaag tatgccttaa gcagaacaaa 2760
   tgtgtttttc tatatagttc cttgccttaa taaatatgta atataaattt aagcaaacgt 2820
   ctattttgta tatttgtaaa ctacaaagta aaatgaacat tttgtggagt ttgtattttg 2880
   catactcaag gtgagaatta agttttaaat aaacctataa tattttatct gaaaaaaaaa 2940
   aaaaaaaaa 2949
<210> 11
   <211> 2121
   <212> DNA
   <213> Homo sapiens
<400> 11
   ctgctcgcgg ccgccaccgc cgggccccgg ccgtccctgg ctcccctcct gcctcgagaa      60
   gggcagggct tctcagaggc ttggcgggaa aaaagaacgg agggagggat cgcgctgagt     120
   ataaaagccg gttttcgggg ctttatctaa ctcgctgtag taattccagc gagaggcaga     180
   gggagcgagc gggcggccgg ctagggtgga agagccgggc gagcagagct gcgctgcggg     240
   cgtcctggga agggagatcc ggagcgaata gggggcttcg cctctggccc agccctcccg     300
   cttgatcccc caggccagcg gtccgcaacc cttgccgcat ccacgaaact ttgcccatag     360
   cagcgggcgg gcactttgca ctggaactta caacacccga gcaaggacgc gactctcccg     420
   acgcggggag gctattctgc ccatttgggg acacttcccc gccgctgcca ggacccgctt     480
   ctctgaaagg ctctccttgc agctgcttag acgctggatt tttttcgggt agtggaaaac     540
   cagcagcctc ccgcgacgat gcccctcaac gttagcttca ccaacaggaa ctatgacctc     600
   gactacgact cggtgcagcc gtatttctac tgcgacgagg aggagaactt ctaccagcag     660
   cagcagcaga gcgagctgca gcccccggcg cccagcgagg atatctggaa gaaattcgag     720
   ctgctgccca ccccgcccct gtcccctagc cgccgctccg ggctctgctc gccctcctac     780
   gttgcggtca cacccttctc ccttcgggga gacaacgacg gcggtggcgg gagcttctcc     840
   acggccgacc agctggagat ggtgaccgag ctgctgggag gagacatggt gaaccagagt     900
   ttcatctgcg acccggacga cgagaccttc atcaaaaaca tcatcatcca ggactgtatg 960
   tggagcggct tctcggccgc cgccaagctc gtctcagaga agctggcctc ctaccaggct 1020
   gcgcgcaaag acagcggcag cccgaacccc gcccgcggcc acagcgtctg ctccacctcc 1080
   agcttgtacc tgcaggatct gagcgccgcc gcctcagagt gcatcgaccc ctcggtggtc 1140
   ttcccctacc ctctcaacga cagcagctcg cccaagtcct gcgcctcgca agactccagc 1200
   gccttctctc cgtcctcgga ttctctgctc tcctcgacgg agtcctcccc gcagggcagc 1260
   cccgagcccc tggtgctcca tgaggagaca ccgcccacca ccagcagcga ctctgaggag 1320
   gaacaagaag atgaggaaga aatcgatgtt gtttctgtgg aaaagaggca ggctcctggc 1380
   aaaaggtcag agtctggatc accttctgct ggaggccaca gcaaacctcc tcacagccca 1440
   ctggtcctca agaggtgcca cgtctccaca catcagcaca actacgcagc gcctccctcc 1500
   actcggaagg actatcctgc tgccaagagg gtcaagttgg acagtgtcag agtcctgaga 1560
   cagatcagca acaaccgaaa atgcaccagc cccaggtcct cggacaccga ggagaatgtc 1620
   aagaggcgaa cacacaacgt cttggagcgc cagaggagga acgagctaaa acggagcttt 1680
   tttgccctgc gtgaccagat cccggagttg gaaaacaatg aaaaggcccc caaggtagtt 1740
   atccttaaaa aagccacagc atacatcctg tccgtccaag cagaggagca aaagctcatt 1800
   tctgaagagg acttgttgcg gaaacgacga gaacagttga aacacaaact tgaacagcta 1860
   cggaactctt gtgcgtaagg aaaagtaagg aaaacgattc cttctaacag aaatgtcctg 1920
   agcaatcacc tatgaacttg tttcaaatgc atgatcaaat gcaacctcac aaccttggct 1980
   gagtcttgag actgaaagat ttagccataa tgtaaactgc ctcaaattgg actttgggca 2040
   taaaagaact tttttatgct taccatcttt tttttttctt taacagattt gtatttaaga 2100
   attgttttta aaaaatttta a 2121
<210> 12
   <211> 2520
   <212> DNA
   <213> Homo sapiens
<400> 12
   ggatggttgt ctattaactt gttcaaaaaa gtatcaggag ttgtcaaggc agagaagaga      60
   gtgtttgcaa aagggggaaa gtagtttgct gcctctttaa gactaggact gagagaaaga     120
   agaggagaga gaaagaaagg gagagaagtt tgagccccag gcttaagcct ttccaaaaaa     180
   taataataac aatcatcggc ggcggcagga tcggccagag gaggagggaa gcgctttttt     240
   tgatcctgat tccagtttgc ctctctcttt ttttccccca aattattctt cgcctgattt     300
   tcctcgcgga gccctgcgct cccgacaccc ccgcccgcct cccctcctcc tctccccccg     360
   cccgcgggcc ccccaaagtc ccggccgggc cgagggtcgg cggccgccgg cgggccgggc     420
   ccgcgcacag cgcccgcatg tacaacatga tggagacgga gctgaagccg ccgggcccgc 480
   agcaaacttc ggggggcggc ggcggcaact ccaccgcggc ggcggccggc ggcaaccaga 540
   aaaacagccc ggaccgcgtc aagcggccca tgaatgcctt catggtgtgg tcccgcgggc 600
   agcggcgcaa gatggcccag gagaacccca agatgcacaa ctcggagatc agcaagcgcc 660
   tgggcgccga gtggaaactt ttgtcggaga cggagaagcg gccgttcatc gacgaggcta 720
   agcggctgcg agcgctgcac atgaaggagc acccggatta taaataccgg ccccggcgga 780
   aaaccaagac gctcatgaag aaggataagt acacgctgcc cggcgggctg ctggcccccg 840
   gcggcaatag catggcgagc ggggtcgggg tgggcgccgg cctgggcgcg ggcgtgaacc 900
   agcgcatgga cagttacgcg cacatgaacg gctggagcaa cggcagctac agcatgatgc 960
   aggaccagct gggctacccg cagcacccgg gcctcaatgc gcacggcgca gcgcagatgc 1020
   agcccatgca ccgctacgac gtgagcgccc tgcagtacaa ctccatgacc agctcgcaga 1080
   cctacatgaa cggctcgccc acctacagca tgtcctactc gcagcagggc acccctggca 1140
   tggctcttgg ctccatgggt tcggtggtca agtccgaggc cagctccagc ccccctgtgg 1200
   ttacctcttc ctcccactcc agggcgccct gccaggccgg ggacctccgg gacatgatca 1260
   gcatgtatct ccccggcgcc gaggtgccgg aacccgccgc ccccagcaga cttcacatgt 1320
   cccagcacta ccagagcggc ccggtgcccg gcacggccat taacggcaca ctgcccctct 1380
   cacacatgtg agggccggac agcgaactgg aggggggaga aattttcaaa gaaaaacgag 1440
   ggaaatggga ggggtgcaaa agaggagagt aagaaacagc atggagaaaa cccggtacgc 1500
   tcaaaaagaa aaaggaaaaa aaaaaatccc atcacccaca gcaaatgaca gctgcaaaag 1560
   agaacaccaa tcccatccac actcacgcaa aaaccgcgat gccgacaaga aaacttttat 1620
   gagagagatc ctggacttct ttttggggga ctatttttgt acagagaaaa cctggggagg 1680
   gtggggaggg cgggggaatg gaccttgtat agatctggag gaaagaaagc tacgaaaaac 1740
   tttttaaaag ttctagtggt acggtaggag ctttgcagga agtttgcaaa agtctttacc 1800
   aataatattt agagctagtc tccaagcgac gaaaaaaatg ttttaatatt tgcaagcaac 1860
   ttttgtacag tatttatcga gataaacatg gcaatcaaaa tgtccattgt ttataagctg 1920
   agaatttgcc aatatttttc aaggagaggc ttcttgctga attttgattc tgcagctgaa 1980
   atttaggaca gttgcaaacg tgaaaagaag aaaattattc aaatttggac attttaattg 2040
   tttaaaaatt gtacaaaagg aaaaaattag aataagtact ggcgaaccat ctctgtggtc 2100
   ttgtttaaaa agggcaaaag ttttagactg tactaaattt tataacttac tgttaaaagc 2160
   aaaaatggcc atgcaggttg acaccgttgg taatttataa tagcttttgt tcgatcccaa 2220
   ctttccattt tgttcagata aaaaaaacca tgaaattact gtgtttgaaa tattttctta 2280
   tggtttgtaa tatttctgta aatttattgt gatattttaa ggttttcccc cctttatttt      2340
   ccgtagttgt attttaaaag attcggctct gtattatttg aatcagtctg ccgagaatcc      2400
   atgtatatat ttgaactaat atcatcctta taacaggtac attttcaact taagttttta      2460
   ctccattatg cacagtttga gataaataaa tttttgaaat atggacactg aaaaaaaaaa      2520
<210> 13
   <211> 15810
   <212> DNA
   <213> Artificial Sequence
<220>
   <223> SeVdp(MKOS)302L
<400> 13
<210> 14
   <211> 15816
   <212> DNA
   <213> Artificial Sequence
<220>
   <223> SeVdp(KOSM)
<400> 14
   accaaacaag agaagaaaca tgtatggaat atataatgaa gttagacagg attttagggt      60
   caaagtatcc accctgagga gcaggttcca gatccttttc tttgctgcca aagttcacga     120
   tggccgggtt gttgagcacc ttcgatacat ttagctctag gaggagcgaa agtattaata     180
   agtcgggagg aggtgctgtt atccccggcc agaggagcac agtctcagtg ttcatactag     240
   gcccaagtgt gactgatgat gcagacaagt tattcattgc aacaaccttc ctagctcact     300
   cattggacac agataagcag cactctcaga gaggagggtt cctcgtctct ctgcttgcca     360
   tggcttacag tagtccagaa ttgtacttga caacaaacgg agtaaacgcc gatgtcaaat      420
   atgtgatcta caacatagag aaagacccta agaggacgaa gacagacgga ttcattgtga      480
   agacgagaga tatggaatat gagaggacca cagaatggct gtttggacct atggtcaaca      540
   agagcccact cttccagggt caacgggatg ctgcagaccc tgacacactc cttcaaatct      600
   atgggtatcc tgcatgccta ggagcaataa ttgtccaagt ctggattgtg ctggtgaagg      660
   ccatcacaag cagcgccggc ttaaggaaag ggttcttcaa caggttagag gcgttcagac      720
   aagacggcac cgtgaaaggt gccttagttt tcactgggga gacagttgag gggataggct      780
   cggttatgag atctcagcaa agccttgtat ctctcatggt tgagaccctt gtgactatga      840
   atactgcaag atctgatctc accacattag agaagaacat ccagatcgtt gggaactaca      900
   tccgagatgc agggctggct tccttcatga acactattaa atatggggtg gagacaaaga      960
   tggcagctct aacgttgtca aacctgaggc ccgatattaa taagcttaga agcctcatag     1020
   acacctacct gtcaaaaggc cccagagctc cctttatctg tatcctcaag gaccctgttc     1080
   atggtgaatt tgctccaggc aattatcctg cactatggag ttacgccatg ggagtcgccg     1140
   tcgtacagaa caagtcaatg cagcagtacg tcacagggag gacatacctt gatatggaaa     1200
   tgttcttact aggacaagcc gtggcaaagg atgctgaatc gaagatcagc agtgccttgg     1260
   aagatgagtt aggagtgacg gatacagcca aggagaggct cagacatcat ctggcaaact     1320
   tgtccggtgg ggatggtgct taccacaaac caacaggcgg tggtgcaatt gaggtagctc     1380
   tagacaatgc cgatatcgac ctggaaacag aagctcatgc ggaccaggac gctaggggtt     1440
   ggggtggaga tagtggtgaa agatgggcac gtcaggtgag tggtggccac tttgtcacac     1500
   tacatggggc tgaacggtta gaggaggaaa ccaatgatga ggatgtatca gacatagaga     1560
   gaagaatagc catgagactc gcagagagac ggcaagagga ttctgcaacc catggagatg     1620
   aaggccgcaa taacggtgtt gatcacgaag aagatgacga tgccgcagca gcagctggga     1680
   taggaggaat ctaggatcat acgaggcctc aaggtacttg atccgcagta agaaaaactt     1740
   agggtgaaag ttcatccacc gatcggctca ggcaaggcca cacccaaccc caccgaccac     1800
   acccagcagt cgagacagcc acggcttcgg ctacacttac cgcatggatc aagatgcctt     1860
   cattcttaaa gaagattctg aagttgagag gaaggcgcca ggaggacgag agtcgctctc     1920
   ggatgttatc ggattcctcg atgctgtcct gtcgaatgaa ccaactgaca tcggagggga     1980
   cagaagctgg ctccacaaca ccatcaacac tccccaagga ccaggctctg ctcatagagc     2040
   caaaagtgag ggcgaaggag aagtctcaac accgtcgacc caagataatc gatcaggtga     2100
   ggagagtaga gtctctggga gaacaagcaa gccagaggca gaagcacatg ctggaaacct     2160
   tgataaacaa aatatacact gggcctttag gggaagaact ggtacaaact ctgtatctca     2220
   ggatctggac gatggaggag actccggaat ccttgaaaat cctccaaatg agagaggata 2280
   tccgagatca ggtattgaag atgaaaacag agagatggct gcgcaccctg ataagagggg 2340
   agaagaccaa gctgaaggac ttccagaaga ggtacgagga ggtacatccc tacctgatga 2400
   aggagaaggt ggagcaagta ataatggaag aagcatggag cctggcagct cacatagtgc 2460
   aagagtaact ggggtcctgg tgattcctag ccccgaactt gaagaggctg tgctacggag 2520
   gaacaaaaga agacctacca acagtgggtc caaacctctt actccagcaa ccgtgcctgg 2580
   cacccggtcc ccaccgctga atcgttacaa cagcacaggg tcaccaccag gaaaaccccc 2640
   atctacacag gatgagcaca tcaactctgg ggacaccccc gccgtcaggg tcaaagaccg 2700
   gaaaccatca atagggactc gctctgtctc agattgtcca gccaacggcc gcccaatcca 2760
   tccgggtata gagaccgact caacaaaaaa gggcatagga gagaacacat catctatgaa 2820
   agatatggct acattgttga cgagtcttgg tgtaatccag tctgctcaag aattcgagtc 2880
   atcccgagac gcgagttatg tgtttgcaag acgtgcccta aagtctgcaa actatgcaga 2940
   gatgacattc aatgtatgcg gcctgatcct ttctgccgag aaatcttccg ctcgtaaggt 3000
   agatgagaac aaacaactgc tcaaacagat ccaagagagc gtggaatcat tccgggatat 3060
   ttacaagaga ttctctgagt atcagaaaga acagaactca ttgctgatgt ccaacctatc 3120
   tacacttcat atcatcacag atagaggtgg caagactgac aacacagact cccttacaag 3180
   gtccccctcc gtttttgcaa aatcaaaaga gaacaagact aaggctacca ggtttgaccc 3240
   atctatggag accttagaag atatgaagta caaaccggac ctaatccgag aggatgaatt 3300
   tagagatgag atccgcaacc cggtgtacca agagagggac acagaaccca gggcctcaaa 3360
   cgcatcacgt ctcttcccct ccaaagagaa gcacacaatg cactctctca ggctcgtcat 3420
   agagagcagt cccctaagca gagctgagaa agcagcatat gtgaaatcat tatccaagtg 3480
   caagacagac caagaggtta aggcagtcat ggaactcgta gaagaggaca tagagtcact 3540
   gaccaactag atcccgggtg aggcatccca ccatcctcag tcacagagag acccaatcta 3600
   ccatcagcat cagccagtaa agattaagaa aaacttaggg tgaaagaaat ttcaccgcta 3660
   gcacctaggt ctgacatggc tgtcagcgac gcgctgctcc catctttctc cacgttcgcg 3720
   tctggcccgg cgggaaggga gaagacactg cgtcaagcag gtgccccgaa taaccgctgg 3780
   cgggaggagc tctcccacat gaagcgactt cccccagtgc ttcccggccg cccctatgac 3840
   ctggcggcgg cgaccgtggc cacagacctg gagagcggcg gagccggtgc ggcttgcggc 3900
   ggtagcaacc tggcgcccct acctcggaga gagaccgagg agttcaacga tctcctggac 3960
   ctggacttta ttctctccaa ttcgctgacc catcctccgg agtcagtggc cgccaccgtg 4020
   tcctcgtcag cgtcagcctc ctcttcgtcg tcgccgtcga gcagcggccc tgccagcgcg 4080
   ccctccacct gcagcttcac ctatccgatc cgggccggga acgacccggg cgtggcgccg 4140
   ggcggcacgg gcggaggcct cctctatggc agggagtccg ctccccctcc gacggctccc 4200
   ttcaacctgg cggacatcaa cgacgtgagc ccctcgggcg gcttcgtggc cgagctcctg 4260
   cggccagaat tggacccggt gtacattccg ccgcagcagc cgcagccgcc aggtggcggg 4320
   ctgatgggca agttcgtgct gaaggcgtcg ctgagcgccc ctggcagcga gtacggcagc 4380
   ccgtcggtca tcagcgtcag caaaggcagc cctgacggca gccacccggt ggtggtggcg 4440
   ccctacaacg gcgggccgcc gcgcacgtgc cccaagatca agcaggaggc ggtctcttcg 4500
   tgcacccact tgggcgctgg accccctctc agcaatggcc accggccggc tgcacacgac 4560
   ttccccctgg ggcggcagct ccccagcagg actaccccga ccctgggtct tgaggaagtg 4620
   ctgagcagca gggactgtca ccctgccctg ccgcttcctc ccggcttcca tccccacccg 4680
   gggcccaatt acccatcctt cctgcccgat cagatgcagc cgcaagtccc gccgctccat 4740
   taccaagagc tcatgccacc cggttcctgc atgccagagg agcccaagcc aaagagggga 4800
   agacgatcgt ggccccggaa aaggaccgcc acccacactt gtgattacgc gggctgcggc 4860
   aaaacctaca caaagagttc ccatctcaag gcacacctgc gaacccacac aggtgagaaa 4920
   ccttaccact gtgactggga cggctgtgga tggaaattcg cccgctcaga tgaactgacc 4980
   aggcactacc gtaaacacac ggggcaccgc ccgttccagt gccaaaaatg cgaccgagca 5040
   ttttccaggt cggaccacct cgccttacac atgaagaggc atttttaaag acgtcgatta 5100
   agaaaaactt agggtgaaag ttcatcgcgg ccgcttgcta gcggttcccc atggcgggac 5160
   acctggcttc ggatttcgcc ttctcgcccc ctccaggtgg tggaggtgat gggccagggg 5220
   ggccggagcc gggctgggtt gatcctcgga cctggctaag cttccaaggc cctcctggag 5280
   ggccaggaat cgggccgggg gttgggccag gctctgaggt gtgggggatt cccccatgcc 5340
   ccccgccgta tgagttctgt ggggggatgg cgtactgtgg gccccaggtt ggagtggggc 5400
   tagtgcccca aggcggcttg gagacctctc agcctgaggg cgaagcagga gtcggggtgg 5460
   agagcaactc cgatggggcc tccccggagc cctgcaccgt cacccctggt gccgtgaagc 5520
   tggagaagga gaagctggag caaaacccgg aggagtccca ggacatcaaa gctctgcaga 5580
   aagaactcga gcaatttgcc aagctcctga agcagaagag gatcaccctg ggatatacac 5640
   aggccgatgt ggggctcacc ctgggggttc tatttgggaa ggtattcagc caaacgacca 5700
   tctgccgctt tgaggctctg cagcttagct tcaagaacat gtgtaagctg cggcccttgc 5760
   tgcagaagtg ggtggaggaa gctgacaaca atgaaaatct tcaggagata tgcaaagcag 5820
   aaaccctcgt gcaggcccga aagagaaagc gaaccagtat cgagaaccga gtgagaggca 5880
   acctggagaa tttgttcctg cagtgcccga aacccacact gcagcagatc agccacatcg 5940
   cccagcagct tgggctcgag aaggatgtgg tccgagtgtg gttctgtaac cggcgccaga 6000
   agggcaagcg atcaagcagc gactatgcac aacgagagga ttttgaggct gctgggtctc 6060
   ctttctcagg gggaccagtg tcctttcctc tggccccagg gccccatttt ggtaccccag 6120
   gctatgggag ccctcacttc actgcactgt actcctcggt ccctttccct gagggggaag 6180
   cctttccccc tgtctccgtc accactctgg gctctcccat gcattcaaac tgaacgcgtc 6240
   agagacctgc aacaatgtct caagcagaca ccacctggca gtcggagcca ccgggtcact 6300
   ccttgtctta aataagaaaa acttagggat aaagtccctt agatctagcc taggcgcatg 6360
   tacaacatga tggagacgga gctgaagccg ccgggcccgc agcaaacttc ggggggcggc 6420
   ggcggcaact ccaccgcggc ggcggccggc ggcaaccaga aaaacagccc ggaccgcgtc 6480
   aagcggccca tgaatgcctt catggtgtgg tcccgcgggc agcggcgcaa gatggcccag 6540
   gagaacccca agatgcacaa ctcggagatc agcaagcgcc tgggcgccga gtggaaactt 6600
   ttgtcggaga cggagaagcg gccgttcatc gacgaggcta agcggctgcg agcgctgcac 6660
   atgaaggagc acccggatta taaataccgg ccccggcgga aaaccaagac gctcatgaag 6720
   aaggataagt acacgctgcc cggcgggctg ctggcccccg gcggcaatag catggcgagc 6780
   ggggtcgggg tgggcgccgg cctgggcgcg ggcgtgaacc agcgcatgga cagttacgcg 6840
   cacatgaacg gctggagcaa cggcagctac agcatgatgc aggaccagct gggctacccg 6900
   cagcacccgg gcctcaatgc gcacggcgca gcgcagatgc agcccatgca ccgctacgac 6960
   gtgagcgccc tgcagtacaa ctccatgacc agctcgcaga cctacatgaa cggctcgccc 7020
   acctacagca tgtcctactc gcagcagggc acccctggca tggctcttgg ctccatgggt 7080
   tcggtggtca agtccgaggc cagctccagc ccccctgtgg ttacctcttc ctcccactcc 7140
   agggcgccct gccaggccgg ggacctccgg gacatgatca gcatgtatct ccccggcgcc 7200
   gaggtgccgg aacccgccgc ccccagcaga cttcacatgt cccagcacta ccagagcggc 7260
   ccggtgcccg gcacggccat taacggcaca ctgcccctct cacacatgtg aggacgtcag 7320
   atctgtatat aataagaaaa acttagggtg aaagtgaggt tgcgcggtat tttagctagc 7380
   ttagacgctg gatttttttc gggtagtgga aaaccagcag cctcccgcga cgatgcccct 7440
   caacgttagc ttcaccaaca ggaactatga cctcgactac gactcggtgc agccgtattt 7500
   ctactgcgac gaggaggaga acttctacca gcagcagcag cagagcgagc tgcagccccc 7560
   ggcgcccagc gaggatatct ggaagaaatt cgagctgctg cccaccccgc ccctgtcccc 7620
   tagccgccgc tccgggctct gctcgccctc ctacgttgcg gtcacaccct tctcccttcg 7680
   gggagacaac gacggcggtg gcgggagctt ctccacggcc gaccagctgg agatggtgac 7740
   cgagctgctg ggaggagaca tggtgaacca gagtttcatc tgcgacccgg acgacgagac 7800
   cttcatcaaa aacatcatca tccaggactg tatgtggagc ggcttctcgg ccgccgccaa 7860
   gctcgtctca gagaagctgg cctcctacca ggctgcgcgc aaagacagcg gcagcccgaa 7920
   ccccgcccgc ggccacagcg tctgctccac ctccagcttg tacctgcagg atctgagcgc 7980
   cgccgcctca gagtgcatcg acccctcggt ggtcttcccc taccctctca acgacagcag 8040
   ctcgcccaag tcctgcgcct cgcaagactc cagcgccttc tctccgtcct cggattctct 8100
   gctctcctcg acggagtcct ccccgcaggg cagccccgag cccctggtgc tccatgagga 8160
   gacaccgccc accaccagca gcgactctga ggaggaacaa gaagatgagg aagaaatcga 8220
   tgttgtttct gtggaaaaga ggcaggctcc tggcaaaagg tcagagtctg gatcaccttc 8280
   tgctggaggc cacagcaaac ctcctcacag cccactggtc ctcaagaggt gccacgtctc 8340
   cacacatcag cacaactacg cagcgcctcc ctccactcgg aaggactatc ctgctgccaa 8400
   gagggtcaag ttggacagtg tcagagtcct gagacagatc agcaacaacc gaaaatgcac 8460
   cagccccagg tcctcggaca ccgaggagaa tgtcaagagg cgaacacaca acgtcttgga 8520
   gcgccagagg aggaacgagc taaaacggag cttttttgcc ctgcgtgacc agatcccgga 8580
   gttggaaaac aatgaaaagg cccccaaggt agttatcctt aaaaaagcca cagcatacat 8640
   cctgtccgtc caagcagagg agcaaaagct catttctgaa gaggacttgt tgcggaaacg 8700
   acgagaacag ttgaaacaca aacttgaaca gctacggaac tcttgtgcgt aagaccggtg 8760
   tcggctttgc tgacactaga gtcatctccg aacatccaca atatctctca gtctcttacg 8820
   tctctcacag tattaagaaa aacccagggt gaatgggaag cttgccatag gtcatggatg 8880
   ggcaggagtc ctcccaaaac ccttctgaca tactctatcc agaatgccac ctgaactctc 8940
   ccatagtcag ggggaagata gcacagttgc acgtcttgtt agatgtgaac cagccctaca 9000
   gactaaagga cgacagcata ataaatatta caaagcacaa aattaggaac ggaggattgt 9060
   cccctcgtca aattaagatc aggtctctgg gtaaggctct tcaacgcaca ataaaggatt 9120
   tagaccgata cacctttgaa ccgtacccaa cctactctca ggaattactt aggcttgata 9180
   taccagagat atgtgacaaa atccgatccg tcttcgcggt ctcggatcgg ctgaccaggg 9240
   agttatctag tgggttccag gatctttggt tgaatatctt caagcaacta ggcaatatag 9300
   aaggaagaga ggggtacgat ccgttgcagg atatcggcac catcccggag ataactgata 9360
   aatacagcag gaatagatgg tataggccat tcctaacttg gttcagcatc aaatatgaca 9420
   tgcggtggat gcagaagacc agaccggggg gacccctcga tacctctaat tcacataacc 9480
   tcctagaatg caaatcatac actctagtaa catacggaga tcttatcatg atactgaaca 9540
   agttgacatt gacagggtat atcctaaccc ctgagctggt cttgatgtat tgtgatgttg 9600
   tagagggaag gtggaatatg tctgctgcag ggcatctaga taagaagtcc attgggataa 9660
   caagcaaagg tgaggaatta tgggaactag tggattccct cttctcaagt cttggagagg 9720
   aaatatacaa tgtcatcgca ctattggagc ccctatcact tgctctcata caactaaatg 9780
   atccagttat acctctacgt ggggcattta tgaggcatgt gttgacagag ctacaggctg 9840
   ttttaacaag tagggacgtg tacacagatg ctgaagcaga cactattgtg gagtcgttac 9900
   tcgccatttt ccatggaacc tctattgatg agaaagcaga gatcttttcc ttctttagga 9960
   catttggcca ccccagctta gaggctgtca ctgccgccga caaggtaagg gcccatatgt 10020
   atgcacaaaa ggcaataaag cttaagaccc tatacgagtg tcatgcagtt ttttgcacta 10080
   tcatcataaa tgggtataga gagaggcatg gcggacagtg gcccccctgt gacttccctg 10140
   atcacgtgtg tctagaacta aggaacgctc aagggtccaa tacggcaatc tcttatgaat 10200
   gtgctgtaga caactataca agtttcatag gcttcaagtt tcggaagttt atagaaccac 10260
   aactagatga agatctcaca atatatatga aagacaaagc actatccccc aggaaggagg 10320
   catgggactc tgtatacccg gatagtaatc tgtactataa agccccagaa tctgaagaga 10380
   cccggcggct tattgaagtg ttcataaatg atgagaattt caacccagaa gaaattatca 10440
   attatgtgga gtcaggagat tggttgaaag acgagaagtt caacatctcg tacagtctca 10500
   aagagaaaga gatcaagcaa gagggtcgtc tattcgcaaa aatgacttat aagatgcgag 10560
   ccgtacaggt gctggcagag acactactgg ctaaaggaat aggagagctg ttcagcgaaa 10620
   atgggatggt taaaggagag atagacctac ttaaaagatt gactactctt tctgtctcag 10680
   gagtccccag gactgattca gtgtacaata actctaaatc atcagagaag agaaacgaag 10740
   gcatgaaaaa gaagaactct ggggggtact gggacgaaaa gaagaggtcc agacatgaat 10800
   tcaaggcaac agattcatca acagacggct atgaaacgtt aagttgcttc ctcacaacag 10860
   acctcaagaa atactgctta aactggagat ttgaaagtac tgcattgttt ggtcagagat 10920
   gcaacgagat atttggcttc aagaccttct ttaactggat gcatccagtc cttgaaaggt 10980
   gtacaatata tgttggggat ccttactgtc cagtcgccga ccggatgcat cgacaactcc 11040
   aggatcatgc agactctggc attttcatac ataatcctag ggggggcata gaaggttact 11100
   gccagaagct gtggacctta atctcaatca gtgcaatcca cctagcagct gtgagagtgg 11160
   gtgtcagggt ctctgcaatg gttcagggtg acaatcaagc tatagccgtg acatcaagag 11220
   tacctgtagc tcagacttac aagcagaaga aaaatcatgt ctataaggag atcaccaaat 11280
   attttggtgc tctaagacac gtcatgtttg atgtagggca cgagctaaaa ttgaacgaga 11340
   ccatcattag tagcaagatg tttgtctata gtaaaagaat atactatgat gggaagattt 11400
   taccacagtg cctgaaagcc ttgaccaggt gtgtattctg gtccgagaca ctggtagatg 11460
   aaaacagatc tgcttgttcg aacatctcaa catccatagc aaaagctatc gaaaatgggt 11520
   attctcctat actaggctac tgcattgcgt tgtataagac ctgtcagcag gtgtgcatat 11580
   cactagggat gactataaat ccaactatca gcccgaccgt aagagatcaa tactttaagg 11640
   gtaagaattg gctgagatgt gcagtgttga ttccagcaaa tgttggagga ttcaactaca 11700
   tgtctacatc tagatgcttt gttagaaata ttggagaccc cgcagtagca gccctagctg 11760
   atctcaaaag attcatcaga gcggatctgt tagacaagca ggtactatac agggtcatga 11820
   atcaagaacc cggtgactct agctttctag attgggcttc agacccttat tcatgtaacc 11880
   tcccgcattc tcagagtata actacgatta taaagaatat cactgctaga tctgtgctgc 11940
   aggaatcccc gaatcctcta ctgtctggtc tcttcaccga gactagtgga gaagaggatc 12000
   tcaacctggc ctcgttcctt atggaccgga aagtcatcct gccgagagtg gctcatgaga 12060
   tcctgggtaa ttccttaact ggagttaggg aggcgattgc agggatgctt gatacgacca 12120
   agtctctagt gagatccagc gttaagaaag gaggattatc atatgggata ttgaggaggc 12180
   ttgtcaatta tgatctattg cagtacgaga cactgactag aactctcagg aaaccggtga 12240
   aagacaacat cgaatatgag tatatgtgtt cagttgagct agctgtcggt ctaaggcaga 12300
   aaatgtggat ccacctaact tacgggagac ccatacatgg gctagaaaca ccagaccctt 12360
   tagagctctt gaggggaaca tttatcgaag gttcagaggt gtgcaagctt tgcaggtctg 12420
   agggagcaga ccccatctat acatggttct atctccctga caatatagac ctggacacgc 12480
   ttacaaacgg atgtccggct ataagaatcc cctattttgg atcagccact gatgaaaggt 12540
   cggaagccca actcgggtat gtaagaaatc taagcaaacc cgcaaaggct gccatccgga 12600
   tagctatggt gtatacgtgg gcctacggga ctgatgagat atcgtggatg gaagccgctc 12660
   ttatagccca aacaagagct aatctgagct tagagaatct aaagctgctg actcctgttt 12720
   caacctccac taatctatct cataggttga aagatacggc aacccagatg aagttctcta 12780
   gtgcaacact agtccgtgca agtcggttca taacaatatc aaatgataac atggcactca 12840
   aagaagcagg ggagtcgaag gatactaatc tcgtgtatca gcagattatg ctaactgggc 12900
   taagcttgtt cgagttcaat atgagatata agaaaggttc cttagggaag ccactgatat 12960
   tgcacttaca tcttaataac gggtgctgta taatggagtc cccacaggag gcgaatatcc 13020
   ccccaaggtc cacattagat ttagagatta cacaagagaa caataaattg atctatgatc 13080
   ctgatccact caaggatgtg gaccttgagc tatttagcaa ggtcagagat gttgtacata 13140
   cagttgacat gacttattgg tcagatgatg aagttatcag agcaaccagt atctgtactg 13200
   caatgacgat agctgataca atgtctcaat tagatagaga caacctaaaa gagatgatcg 13260
   cgctagtaaa tgacgatgat gtcaacagcc tgattactga gtttatggtg attgatgttc 13320
   ctttattttg ctcaacgttc gggggtattc tagtcaatca gtttgcatac tcactctacg 13380
   gcttaaacat cagaggaagg gaagaaatat ggggacatgt agtccggatt cttaaagata 13440
   cctcccacgc agttctaaaa gtcttatcta atgctctatc tcatcccaaa atcttcaaac 13500
   gattctggaa tgcaggtgtc gtggaacctg tgtatgggcc taacctctca aatcaggaca 13560
   agatactctt ggccctctct gtctgtgaat attctgtgga tctattcatg cacgattggc 13620
   aagggggtgt accgcttgag atctttatct gtgacaatga cccagatgtg gccgacatga 13680
   ggaggtcctc tttcttggca agacatcttg catacctatg cagcgtggca gagatatcta 13740
   gggatgggcc aagattagaa tcaatgaact ctctagagag gctcgagtca ctaaagagtt 13800
   acctggaact cacatttctt gatgacccgg tactgaggta cagtcagttg actggcctag 13860
   tcatcaaagt attcccatct actttgacct atatccggaa gtcatctata aaagtgttaa 13920
   ggacaagagg tataggagtc cctgaagtct tagaagattg ggatcccgag gcagataatg 13980
   cactgttaga tggtatcgcg gcagaaatac aacagaatat tcctttggga catcagacta 14040
   gagccccttt ttgggggttg agagtatcca agtcacaggt actgcgtctc cgggggtaca 14100
   aggagatcac aagaggtgag ataggcagat caggcgttgg tctgacgtta ccattcgatg 14160
   gaagatatct atctcaccag ctgaggctct ttggcatcaa cagtactagc tgcttgaaag 14220
   cacttgaact tacctaccta ttgagcccct tagttgacaa ggataaagat aggctatatt 14280
   taggggaagg agctggggcc atgctttcct gttatgacgc tactcttggc ccatgcatca 14340
   actattataa ctcaggggta tactcttgtg atgtcaatgg gcagagagag ttaaatatat 14400
   atcctgctga ggtggcactg gtgggaaaga aattaaacaa tgttactagt ctgggtcaaa 14460
   gagttaaagt gttattcaac gggaatcctg gctcgacatg gattggaaat gatgagtgtg 14520
   aggctttgat ttggaatgaa ttgcagaata gctcgatagg cctagtccac tgtgacatgg 14580
   agggaggaga tcataaggat gatcaagttg tactgcatga gcattacagt gtaatccgga 14640
   tcgcgtatct ggtgggggat cgagacgttg tgcttataag caagattgct cctaggctgg 14700
   gcacggattg gaccaggcag ctcagcctat atctgagata ctgggacgag gttaacctaa 14760
   tagtgcttaa aacatctaac cctgcttcca cagagatgta tctcctatcg aggcatccca 14820
   aatctgacat tatagaggac agcaagacgg tgttagctag tctcctccct ttgtcaaaag 14880
   aagatagcat caagatagaa aagtggatct taatagagaa ggcaaaggct cacgaatggg 14940
   ttactcggga attgagagaa ggaagctctt catcagggat gcttagacct taccatcaag 15000
   cactgcagac gtttggcttt gaaccaaact tgtataaatt gagcagagat ttcttgtcca 15060
   ccatgaacat agctgataca cacaactgca tgatagcttt caacagggtt ttgaaggata 15120
   caatcttcga atgggctaga ataactgagt cagataaaag gcttaaacta actggtaagt 15180
   atgacctgta tcctgtgaga gattcaggca aattgaagac agtttctaga agacttgtgc 15240
   tatcttggat atctttatct atgtccacaa gattggtaac tgggtcattc cctgaccaga 15300
   agtttgaagc aagacttcaa ttgggaatag tttcattatc atcccgtgaa atcaggaacc 15360
   tgagggttat cacaaaaact ttattagacc ggtttgagga tattatacat agtataacgt 15420
   acagattcct caccaaagaa ataaagattt tgatgaagat tttaggggca gtcaagatgt 15480
   tcggggccag gcaaaatgaa tacacgaccg tgattgatga tggatcactg ggtgatatcg   15540
   agccatatga cagctcgtaa taccggttat caccaaaaca tggaagcact tacgattcac   15600
   caaaacatgg aagcacttag gtacctcacc aaaacatgga agcacttacg attcaccaaa   15660
   acatggaagc acttaaccgg ttccctatcg tgcagaacga tcgaagctcc gcggtacctg   15720
   gaagtcttgg actgatccat atgacaatag taagaaaaac ttacaagaag acaagaaaat   15780
   ttaaaagaat acatatctct taaactcttg tctggt                             15816